(19) Europäisches Patentamt
European Patent Office
Office européen des brevets

(11) EP 4 806 879 A1

(12) **EUROPEAN PATENT APPLICATION**
published in accordance with Art. 153(4) EPC

(43) Date of publication:
**16.09.2026 Bulletin 2026/38**

(21) Application number: **24888562.6**

(22) Date of filing: **25.10.2024**

(51) International Patent Classification (IPC):
***C07C 45/35*** (2006.01) ***B01J 23/887*** (2006.01)
***C07B 61/00*** (2006.01) ***C07C 47/22*** (2006.01)
***C07C 51/215*** (2006.01) ***C07C 51/235*** (2006.01)
***C07C 57/05*** (2006.01)

(52) Cooperative Patent Classification (CPC):
**B01J 23/887; C07B 61/00; C07C 45/35; C07C 47/22; C07C 51/215; C07C 51/235; C07C 57/04**

(86) International application number:
**PCT/JP2024/038105**

(87) International publication number:
**WO 2025/100265 (15.05.2025 Gazette 2025/20)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **09.11.2023 JP 2023191217**

(71) Applicant: **Nippon Kayaku Kabushiki Kaisha**
**Tokyo 100-0005 (JP)**

(72) Inventors:
- **OKUMURA, Shigeki**
  **Sanyoonoda-shi, Yamaguchi 757-8686 (JP)**
- **KAWAMURA, Tomoyuki**
  **Tokyo 100-0005 (JP)**

(74) Representative: **Gille Hrabal Partnerschaftsgesellschaft mbB**
**Patentanwälte**
**Brucknerstraße 20**
**40593 Düsseldorf (DE)**

(54) **METHOD FOR PRODUCING UNSATURATED ALDEHYDE OR UNSATURATED CARBOXYLIC ACID**

(57) The method for producing an unsaturated aldehyde or an unsaturated carboxylic acid of the present disclosure is a method of partially oxidizing an alkene to produce a corresponding unsaturated aldehyde or unsaturated carboxylic acid using a fixed-bed multi-tubular reactor and is characterized in that the production parameter T satisfies the following formula (I).

$$T \leq 20.5 \quad (I)$$

EP 4 806 879 A1

## Description

TECHNICAL FIELD

The present invention relates to a method of subjecting an alkene to catalytic gas-phase oxidation with molecular oxygen or a molecular oxygen-containing gas to produce a corresponding unsaturated aldehyde or unsaturated carboxylic acid.

BACKGROUND ART

A method for producing a corresponding unsaturated aldehyde and/or unsaturated carboxylic acid using propylene, isobutylene, tertiary butyl alcohol, or the like as a raw material is widely practiced industrially (for example, PTL 1). PTL 1 relates to a catalyst for improving the yield of an unsaturated aldehyde and/or an unsaturated carboxylic acid which is the target product, but a plurality of techniques relating not only to the catalyst itself but also to a method for packing the catalyst have also been disclosed as described below.

For example, PTL 2 discloses a technique using catalysts whose activities are adjusted by changing the occupation volumes of the shaped catalysts and the calcination temperatures of the catalysts. This is a method in which catalysts containing specific inert carriers are packed in such a manner that the activity level rises from the reaction gas inlet side toward the outlet side in order to suppress generation of locally high-temperature spots (hot spots) in the catalyst layers. PTL 3 discloses a technique for obtaining a target product with a high yield and a high activity by packing the catalysts in such a manner that the composition ratio of the active components of the catalyst varies from the reaction gas inlet side toward the outlet side. Further, PTL 4 discloses a technique for suppressing hot spots by defining the fill length ratio of the catalyst layers packed in two layers to obtain an unsaturated aldehyde in a high yield. PTL 5 discloses a technique for suppressing hot spot temperatures and achieving a high yield by defining the reaction conditions and the catalysts to control the temperature change of the hot spots per reaction bath temperature.

CITATION LIST

PATENT LITERATURE

PTL 1: JP7209901B
PTL 2: JP2001-328951A
PTL 3: JP6694884B
PTL 4: JP6912153B
PTL 5: JP6199972B


SUMMARY OF INVENTION

TECHNICAL PROBLEM

However, for stable operation in a plant, the techniques related to the filling methods disclosed above are considered insufficient. This is because, in the case of a filling method in which the yield of the target product varies sharply with a change in the reaction bath temperature, which is a control factor during the plant operation, stable operation of the plant becomes difficult for the three reasons listed in (1) to (3) below. That is, (1) because the methods for producing an unsaturated aldehyde and/or an unsaturated carboxylic acid described above are exothermic reactions, it is known to use a multi-tubular flow reactor in an actual plant. However, the reaction bath temperatures in the reaction tubes are not necessarily spatially even, and there is a problem of a temperature difference in each reaction tube. In this case, since the calorific values of the reaction tubes are different from each other, the hot spot temperatures (peak temperatures) are also different, and the conversion rates of the raw materials are also different. As a result, the yields of the target product of the reaction tubes are also different from each other, and thus the target product cannot be stably obtained. (2) The reaction bath temperature is controlled by measuring the temperature of the heating medium in the reactor with a thermocouple and heating the heating medium with a heater, but the feedback of the temperature control of the heater is not always given perfectly. For example, hunting due to poor feedback, feedback failure due to unintended disturbance or component failure, and the like occur, and thus the reaction bath temperature is not always constant and may vary. As a result, the yield of the target product also varies sharply, and the target product cannot be stably obtained over time. (3) As a guideline for operation in an actual plant, there is a method of directly measuring the yield of the target product and the conversion rate of

the raw material in the plant with an analytical instrument such as gas chromatography. When the yield of the target product or the conversion rate of the raw material is not in the desired range, the plant operator makes fine adjustment of the reaction bath temperature. Here, when the yield of the target product varies sharply with a change in the reaction bath temperature, the plant operator has to be careful in the fine adjustment of the reaction bath temperature, and unnecessary load is applied to reproduction confirmation and decision making of the above analysis.

In order to overcome the problems in (1) to (3) above, a method for packing catalysts which exhibits a higher yield of a target product in a wider reaction bath temperature range than those in the related art has been required, but such a method has not been disclosed so far. In particular, when the target product is an unsaturated carboxylic acid in a method of subjecting an alkene to catalytic gas-phase oxidation with molecular oxygen or a molecular oxygen-containing gas to produce a corresponding unsaturated aldehyde or unsaturated carboxylic acid, it can be said that a method for packing catalysts which exhibits a higher yield of the target product in a wider reaction bath temperature range than those in the related art has not been disclosed. Therefore, the present inventors have intensively studied and found that the yield of the target product is stably obtained over a longer period of time in a wider reaction bath temperature range than those in the related art by designing the filling method in such a manner that a parameter T which integrates the temperature sensitivity parameter, the dilution rate, the fill length ratio, and the space velocity of the raw material of the catalyst satisfies a specific numerical range, and the invention has been thus completed.

SOLUTION TO PROBLEM

As a result of intensive studies on the present state and the problems described above, the present inventors have focused on packing catalysts so as to widen the reaction bath temperature range (hereinafter, referred to as an operation window) in which the sum of the yields of an unsaturated aldehyde and an unsaturated carboxylic acid, in particular the sum of the yields of acrolein and acrylic acid (effective yield) is stable when carrying out reaction with filling having two or more divided catalyst layers in a method of partially oxidizing an alkene to produce a corresponding unsaturated aldehyde or unsaturated carboxylic acid using a fixed-bed multi-tubular reactor. Here, it has been found that by optimally setting the slope of the raw material conversion rate with respect to the reaction bath temperature (hereinafter, referred to as temperature sensitivity), the fill length ratio, and the dilution rate of the catalyst of each layer in accordance with the load of the raw material which is the reaction condition, the operation window can be widened (that is, a high effective yield can be stably achieved in a wider reaction bath temperature range), the effective yield itself is also increased, and stable plant operation with a higher yield is possible.

That is, the present invention relates to the following 1) to 8).

1) A method for producing an unsaturated aldehyde or an unsaturated carboxylic acid which is a method of partially oxidizing an alkene to produce a corresponding unsaturated aldehyde or unsaturated carboxylic acid using a fixed-bed multi-tubular reactor and in which the production parameter T satisfies the following formula (I).

$$T \leq 20.5 \quad (I)$$

in which,

T is calculated from the average filling parameter Savg and the space velocity SVo of the raw material according to the equation (IV).

$$T\ (\%\cdot hr/°C) = Savg\ (\%/°C) \div SVo\ (/hr) \times 10000 \quad (IV)$$

Savg is calculated from the arithmetic mean of the filling parameters Si of all the catalyst-packed layers according to the equation (III).

$$Savg\ (\%/°C) = (S1\ (\%/°C) + \cdots + Sn\ (\%/°C)) \div n \quad (III)$$

Si is calculated from the temperature sensitivity parameter CBi, the dilution rate di, and the fill length ratio Li of the catalyst of the i-th layer according to the equation (II).

$$Si\ (\%/°C) = CBi\ (\%/°C) \times di\ (\%) \div 100 \times Li \quad (II)$$

CBi is a parameter relating to the temperature sensitivity of the catalyst of the i-th layer and is the rate of the change in the raw material conversion rate with the reaction bath temperature. In addition, n is the total number of the

catalyst layers in the reaction tube. The index i is a natural number of 1 to n. The catalyst layers do not include any inert layer which is filled intentionally only with an inert substance.

2) The method for producing an unsaturated aldehyde or an unsaturated carboxylic acid according to 1) above in which the production parameter T satisfies the following formula (V).

$$9.5 \leq T \leq 16.5 \quad (V)$$

3) The method for producing an unsaturated aldehyde or an unsaturated carboxylic acid according to 1) or 2) above in which the average filling parameter Savg satisfies the following formula (VI).

$$0.090 \leq Savg \leq 0.30 \quad (VI)$$

4) The method for producing an unsaturated aldehyde or an unsaturated carboxylic acid according to any one of 1) to 3) above in which the catalytically active component contained in the catalyst layer closest to the inlet of the raw material gas for reaction has a composition represented by the following formula (1-1).

$$Mo_{a1}Bi_{b1}Ni_{c1}Co_{d1}Fe_{e1}X_{f1}Cs_{g1}Z_{h1}O_{i1} \quad (1\text{-}1)$$

In the formula (1-1), Mo, Bi, Ni, Co, Fe, and Cs respectively represent molybdenum, bismuth, nickel, cobalt, iron, and cesium, and X is at least one element selected from tungsten, antimony, tin, zinc, chromium, manganese, magnesium, silicon, aluminum, cerium, and titanium. Z belongs to Group 1 to Group 16 in the periodic table and means at least one element selected from elements other than the above Mo, Bi, Ni, Co, Fe, Cs, and X, and a1, b1, c1, d1, e1, f1, g1, h1, and i1 respectively represent the numbers of atoms of molybdenum, bismuth, nickel, cobalt, iron, X, Cs, Z, and oxygen. When $a1 = 12$, $0 < b1 \leq 7.0$, $0 \leq c1 \leq 10$, $0 < d1 \leq 10$, $0 < c1 + d1 \leq 20$, $0 < e1 \leq 5.0$, $0 \leq f1 \leq 2.0$, $0 < g1 \leq 3.0$, and $0 \leq h1 \leq 5.0$ are satisfied, and i1 is a value determined by the oxidation state of each element.

5) The method for producing an unsaturated aldehyde or an unsaturated carboxylic acid according to any one of 1) to 4) above in which the catalytically active component contained in the catalyst layer closest to the outlet of the raw material gas for reaction has a composition represented by the following formula (1-2).

$$Mo_{a2}Bi_{b2}Ni_{c2}Co_{d2}Fe_{e2}X_{f2}K_{g2}Z_{h2}O_{i2} \quad (1\text{-}2)$$

In the formula (1-2), Mo, Bi, Ni, Co, Fe, and K respectively represent molybdenum, bismuth, nickel, cobalt, iron, and potassium, and X is at least one element selected from tungsten, antimony, tin, zinc, chromium, manganese, magnesium, silicon, aluminum, cerium, and titanium. Z belongs to Group 1 to Group 16 in the periodic table and means at least one element selected from elements other than the above Mo, Bi, Ni, Co, Fe, K, and X, and a2, b2, c2, d2, e2, f2, g2, h2, and i2 respectively represent the numbers of atoms of molybdenum, bismuth, nickel, cobalt, iron, X, K, Z, and oxygen. When $a2 = 12$, $0 < b2 \leq 7.0$, $0 \leq c2 \leq 10$, $0 < d2 \leq 10$, $0 < c2 + d2 \leq 20$, $0 < e2 \leq 5.0$, $0 \leq f2 \leq 2.0$, $0 \leq g2 \leq 3.0$, and $0 \leq h2 \leq 5.0$ are satisfied, and i2 is a value determined by the oxidation state of each element.

6) The method for producing an unsaturated aldehyde or an unsaturated carboxylic acid according to any one of 1) to 5) above in which the plant is operated at a reaction bath temperature of 310°C or higher and 390°C or lower.

7) The method for producing an unsaturated aldehyde or an unsaturated carboxylic acid according to any one of 1) to 6) above in which the volume ratio of the alkene and the oxygen (oxygen/alkene) of the raw material gas for reaction is 1.0 or more and 1.9 or less.

8) The method for producing an unsaturated aldehyde or an unsaturated carboxylic acid according to any one of 1) to 7) above

in which n layers (n is 2 or more) of catalyst layers are provided in the gas flow direction of the reaction tube and L/Ln is 0.1 or more and 1.0 or less, where the sum of the fill lengths of the first to n-1-th catalyst layers counted from the reaction gas inlet side is L, and the fill length of the n-th catalyst layer counted from the reaction gas inlet side is Ln.

ADVANTAGEOUS EFFECTS OF INVENTION

According to the invention, in the case of producing a corresponding unsaturated aldehyde and unsaturated carboxylic acid using an alkene or an alcohol capable of producing an alkene by an intramolecular dehydration reaction thereof as a raw material, the target product can be obtained in a high yield in a wide reaction bath temperature range even

in an industrial plant.

DESCRIPTION OF EMBODIMENTS

The method for producing an unsaturated carboxylic acid of the invention is characterized in that the production parameter T satisfies the following formula (I).
[Formula 1]

$$T \leq 20.5 \quad (I)$$

The lower limit of the production parameter T may be 0 and is 0.5, 1.0, 1.5, 2.0, 2.5, 3.0, 3.5, 4.0, 4.5, 5.0, 5.5, 6.0, 6.5, 7.0, 7.5, 8.0, 8.5, 9.0, 9.5, 10.0, 10.5, or 11.0 in order of preference. The upper limit of the production parameter T is 19.0, 18.5, 18.0, 17.5, 17.0, 16.5, 16.0, 15.5, 15.0, 14.5, or 14.3 in order of further preference. That is, the production parameter T is preferably 0 or more and 20.5 or less, more preferably 0.5 or more and 20.5 or less, more preferably 1.0 or more and 20.5 or less, more preferably 1.5 or more and 20.5 or less, more preferably 2.0 or more and 20.5 or less, more preferably 2.5 or more and 20.5 or less, more preferably 3.0 or more and 20.5 or less, more preferably 3.5 or more and 20.5 or less, more preferably 4.0 or more and 20.5 or less, more preferably 4.5 or more and 20.5 or less, more preferably 5.0 or more and 20.5 or less, more preferably 5.5 or more and 20.5 or less, more preferably 6.0 or more and 19.0 or less, more preferably 6.5 or more and 18.5 or less, more preferably 7.0 or more and 18.0 or less, more preferably 7.5 or more and 17.5 or less, more preferably 8.0 or more and 17.0 or less, more preferably 8.5 or more and 16.5 or less, more preferably 9.0 or more and 16.0 or less, more preferably 9.5 or more and 15.5 or less, more preferably 10.0 or more and 15.0 or less, and more preferably 10.5 or more and 14.5 or less, and the most preferable range is 11.0 or more and 14.3 or less.

Heretofore, there has been a knowledge that defines the rate of change in the hot spot temperature with the reaction bath temperature from the viewpoint of yield improvement and safe operation of the plant (PTL 5 and the like), but there is no knowledge regarding a catalyst design and a packing design that stably achieve a high effective yield in a wide reaction temperature range, and it can be considered that the invention has elucidated for the first time. When the catalysts are packed in such a manner that the conditions of the invention are satisfied, the operation window can be widened, and safe and stable plant operation with a higher yield is achieved. Moreover, by suppressing the runaway reaction, thermal stress of the catalysts is also prevented, and the life is also expected to be prolonged. More specifically, due to a slight change in the reaction bath temperature or a difference in the reaction bath temperature among the multiple reaction tubes, the hot spots varies sharply on the catalyst on the outlet side, at which the activity is the highest, thereby preventing thermal runaway and resulting damage or explosion of the reaction tubes. When the operation window is wide, an advantage is that the effective yield can be simply stably maintained high at a lower reaction bath temperature and thus the problems described above can be solved. On the other hand, depending on the conditions of the plant and the constraints of the equipment, it can be sometimes preferable to increase the reaction bath temperature to some extent because the relative ratio of acrylic acid to acrolein can be increased and because the load on the subsequent catalyst can be reduced in the partial oxidation reaction of acrylic acid from acrolein which is the subsequent reaction. That is, as another aspect of the advantages of the technique of the invention, a filling method in which a high acrylic acid yield is achieved stably in a wide range of reaction bath temperature, considering the load on the subsequent catalyst, can be provided.

Hereinafter, a method for calculating the parameter T of the invention will be described.

[Calculation of CBi]

The temperature sensitivity parameter CBi of the catalyst of the invention is defined as the rate of the change in the raw material conversion rate (x) with the reaction bath temperature (BT) in the temperature range of 340°C to 360°C of the reaction bath temperature of the catalyst in the i-th layer. Here, for convenience, all the catalyst-packed layers (however, any inert layer that is filled intentionally only with an inert carrier is excluded from the catalyst layers of the invention) are numbered as the first layer, the second layer, ... from the catalyst layer closest to the inlet, and the corresponding CBi is named as CB1, CB2, ... (in the invention, the other parameters are defined in the same manner). Moreover, the total number of the layers of the catalysts is defined as n. The CBi is defined more specifically as follows. The catalyst of the i-th layer is packed into a differential system reactor. The raw material gas conversion rate x (340°C) (unit: %) obtained at a reaction bath temperature (hereinafter sometimes referred to as BT) of 340°C and the raw material conversion rate x (360°C) (unit: %) obtained at BT 360°C are plotted, and the slope of the straight line connecting these points is defined as CBi. That is, CBi is represented by the following equation.

$$CBi\ (\%/°C) = (x\ (360°C)\ (\%) - x\ (340°C))\ (\%)/20\ (°C)$$

Here, the temperature sensitivity parameter of a catalyst calculated at a stage where the layer number to be filled is not determined is simply referred to as CB of the catalyst for convenience in the invention. Moreover, in the invention, the above raw material gas conversion rate x is calculated by the following method. A reaction tube having an inner diameter of 28.4 mm is filled with 4 g of a catalyst in a state of being diluted with an inert substance so as not to generate any hot spot, and reaction is carried out at a molar ratio of propylene:oxygen:nitrogen:water = 1:1.7:6.4:3.0 at a space velocity (GHSV) of propylene set at 400 $hr^{-1}$ at reaction bath temperatures of 340°C and 360°C. The raw material gas conversion rate x is calculated by the following equation using the calibrated gas chromatography.

A calculation formula for a case where the raw material gas is propylene is shown below. The number of moles of reacted propylene is the value obtained by subtracting the number of moles of propylene remaining after the reaction from the number of moles of propylene supplied.

$$\text{Propylene conversion rate x (mol\%)} = (\text{number of moles of reacted propylene/number of moles of propylene supplied}) \times 100$$

[Calculation of Savg]

Next, a method for calculating the average filling parameter Savg of the invention will be described below. First, when the catalyst is diluted with an inert carrier in each layer, the dilution rate di is set as follows. That is, di is the mass percentage (unit: mass%) of the catalyst compact in the i-th catalyst layer, and di is 100 in an undiluted catalyst layer. Next, the fill length ratio Li is set. The fill length ratio Li is the ratio of the fill length of the i-th catalyst layer to the fill length of all the catalyst-packed layers (excluding any inert layer). For example, when the fill length on the inlet side is 20 cm and the fill length on the outlet side is 80 cm in two-layer packed catalysts, L1 (inlet side) is 0.20. In the case of one-layer filling, Li is 1.00. From CBi, di, and Li, the parameter Si of the invention in the i-th catalyst layer is calculated according to the following equation (II).
[Equation II]

$$\text{Si (\%/°C)} = \text{CBi (\%/°C)} \times \text{di (\%)} \div 100 \times \text{Li} \quad \text{equation (II)}$$

Savg is calculated by arithmetically averaging the parameters Si (first layer: S1, second layer: S2 . . .) of the layers which are calculated in this way, according to the following equation (III). Savg is a filling parameter determined by the catalyst type and the filling package, and Savg is S1 when a single catalyst layer is packed.
[Equation III]

$$\text{Savg (\%/°C)} = (\text{S1 (\%/°C)} + ... + \text{Sn (\%/°C)}) \div \text{n} \quad \text{(III)}$$

Here, the number n of the catalyst layers is preferably 2 to 5, further preferably 2 to 4, particularly preferably 2 or 3, and most preferably 3.

[Calculation of T]

Finally, the production parameter T is calculated from Savg and the space velocity SVo of the raw material according to the following equation (IV).
[Equation IV]

$$\text{T (\%·hr/°C)} = \text{Savg (\%/°C)} \div \text{SVo (/hr)} \times 10000 \quad \text{(IV)}$$

Here, the lower limit of Savg is preferably 0.090, 0.100, 0.150, 0.170, 0.190, 0.200, or 0.220 in this order and is particularly preferably 0.240. Moreover, the upper limit of Savg is preferably 0.300, 0.290, 0.280, or 0.275 in this order and is particularly preferably 0.270. That is, Savg is preferably 0.090 or more and 0.300 or less, more preferably 0.100 or more and 0.300 or less, more preferably 0.150 or more and 0.300 or less, more preferably 0.170 or more and 0.300 or less, more preferably 0.190 or more and 0.290 or less, more preferably 0.200 or more and 0.280 or less, more preferably 0.220 or more and 0.275 or less, and most preferably 0.240 or more and 0.270 or less.

[Calculation of A, A1, and A2]

Hereinafter, the operation window of the invention will be more specifically defined. The reaction bath tempera-

ture at which the total yield of the unsaturated aldehyde and the unsaturated carboxylic acid (hereinafter referred to as effective yield) is the highest is defined as A (°C), and the reaction bath temperatures at which the yield is 1.0% lower than the maximum are defined as A1 (°C) and A2 (°C). A is higher than A1, and A2 is higher than A. (A2-A1) is defined as the operation window in the narrow sense of the invention. (A2-A1) is preferably 23°C or more. Here, (A2-A1) is the reaction bath temperature range in which the effective yield is stable (the operation window of the invention), and as (A2-A1) is larger, the unsaturated aldehyde and the unsaturated carboxylic acid can be obtained at a wider reaction bath temperature, which is desirable. Here, A1 and A2 do not have to be calculated from the relationship between the measured effective yield and BT, and a method of calculating from the interpolation or the extrapolation of a series of data obtained by measuring the unsaturated aldehyde and unsaturated carboxylic acid yields with changing the BT may be used (in this case, the extrapolation value is calculated from the measured data of at least three points by linear approximation by the least squares method). In addition, in the case where there are two or more measurement points at which the yield is the highest in the data measured with changing the BT, the measurement point having a higher selectivity is set as A in the invention. The reason why the effective yield decreases in the vicinity of A1 is that the reaction rate of the raw material decreases, and the reason why the effective yield decreases in the vicinity of A2 is due to the generation of decomposition products and the like such as carbon dioxide and acetaldehyde represented by the sequential oxidation reaction of the unsaturated aldehyde and the unsaturated carboxylic acid.

[Catalyst]

The catalyst used in the invention is preferably a catalyst having a composition represented by the following formula (1).
[Formula 1]

$$Mo_aBi_bNi_cCo_dFe_eX_fY_gZ_hO_i \qquad (1)$$

In the formula (1) above, Mo, Bi, Ni, Co, and Fe respectively represent molybdenum, bismuth, nickel, cobalt, and iron, and X is at least one element selected from tungsten, antimony, tin, zinc, chromium, manganese, magnesium, silicon, aluminum, cerium, and titanium. Y is at least one element selected from sodium, potassium, cesium, rubidium, and thallium, and Z belongs to Group 1 to Group 16 in the periodic table and means at least one element selected from elements other than the above Mo, Bi, Ni, Co, Fe, X, and Y. a, b, c, d, e, f, g, h, and i respectively represent the numbers of atoms of molybdenum, bismuth, nickel, cobalt, iron, X, Y, Z, and oxygen. When $a = 12$, $0 \leq b \leq 7.0$, $0 \leq c \leq 10$, $0 \leq d \leq 10$, $0 \leq c + d \leq 20$, $0 \leq e \leq 5.0$, $0 \leq f \leq 2.0$, $0 \leq g \leq 3.0$, and $0 \leq h \leq 5.0$ are satisfied, and i is a value determined by the oxidation state of each element.

In the above formula (1), when a = 12, preferable ranges of b to h are as follows.

The lower limit of b is 0.10, 0.20, 0.30, 0.40, 0.50, 0.60, or 0.70 in order of preference, and the upper limit thereof is 6.0, 5.0, 4.0, 3.0, 2.0, 1.8, 1.5, 1.2, or 1.0 in order of preference. That is, b is preferably 0.10 or more and 6.0 or less, more preferably 0.10 or more and 5.0 or less, more preferably 0.10 or more and 4.0 or less, more preferably 0.20 or more and 3.0 or less, more preferably 0.30 or more and 2.0 or less, more preferably 0.40 or more and 1.8 or less, more preferably 0.50 or more and 1.5 or less, and more preferably 0.60 or more and 1.2 or less, and the most preferable range is 0.70 or more and 1.0 or less.

The lower limit of c is 0.20, 0.50, 0.80, 1.0, 1.2, 1.5, 1.6, or 1.7 in order of preference, and the upper limit thereof is 8.0, 7.0, 6.0, 5.0, 4.0, 3.5, 3.4, or 3.3 in order of preference. That is, c is preferably 0.20 or more and 8.0 or less, more preferably 0.50 or more and 7.0 or less, more preferably 0.80 or more and 6.0 or less, more preferably 1.0 or more and 5.0 or less, more preferably 1.2 or more and 4.0 or less, more preferably 1.5 or more and 3.5 or less, and more preferably 1.6 or more and 3.4 or less, and the most preferable range is 1.7 or more and 3.3 or less.

The lower limit of d is 1.0, 2.0, 3.0, 4.0, or 5.0 in order of preference, and the upper limit thereof is 9.5, 9.0, 8.5, 8.0, 7.5, 7.0, 6.8, 6.7, or 6.6 in order of preference. That is, d is preferably 1.0 or more and 9.5 or less, more preferably 1.0 or more and 9.0 or less, more preferably 1.0 or more and 8.5 or less, more preferably 1.0 or more and 8.0 or less, more preferably 1.0 or more and 7.5 or less, more preferably 2.0 or more and 7.0 or less, more preferably 3.0 or more and 6.8 or less, and more preferably 4.0 or more and 6.7 or less, and the most preferable range is 5.0 or more and 6.6 or less.

The lower limit of c + d is 1.2, 2.0, 4.0, 6.0, 6.5, 6.6, or 6.7 in order of preference, and the upper limit thereof is 20.0, 15.0, 12.5, 11.0, 10.2, 10.1, 10.0, or 9.9 in order of preference. That is, c + d is preferably 1.2 or more and 20.0 or less, more preferably 1.2 or more and 15.0 or less, more preferably 2.0 or more and 12.5 or less, more preferably 4.0 or more and 11.0 or less, more preferably 6.0 or more and 10.2 or less, more preferably 6.5 or more and 10.1 or less, and more preferably 6.6 or more and 10.0 or less, and the most preferable range is 6.7 or more and 9.9 or less.

The lower limit of e is 0.10, 0.20, 0.50, 0.80, 1.0, 1.5, or 1.6 in order of preference, and the upper limit thereof is 4.5, 4.0, 3.5, 3.0, 2.5, 2.4, 2.3, or 2.2 in order of preference. That is, e is preferably 0.10 or more and 4.5 or less, more preferably 0.10 or more and 4.0 or less, more preferably 0.20 or more and 3.5 or less, more preferably 0.50 or more and 3.0 or less,

more preferably 0.80 or more and 2.5 or less, more preferably 1.0 or more and 2.4 or less, and more preferably 1.5 or more and 2.3 or less, and the most preferable range is 1.6 or more and 2.2 or less.

The upper limit of f is 1.8, 1.5, 1.0, 0.80, or 0.50 in order of preference, and the lower limit thereof is preferably 0. That is, a more preferable range of f is 0 or more and 0.50 or less, and 0 is most preferable.

The lower limit of g is 0.010, 0.020, or 0.030 in order of preference, and the upper limit thereof is 2.0, 1.0, 0.50, 0.40, 0.30, 0.20, 0.15, or 0.090 in order of preference. That is, g is preferably 0.010 or more and 2.0 or less, more preferably 0.010 or more and 1.0 or less, more preferably 0.010 or more and 0.50 or less, more preferably 0.010 or more and 0.40 or less, more preferably 0.010 or more and 0.30 or less, more preferably 0.010 or more and 0.20 or less, and more preferably 0.020 or more and 0.10 or less, and the most preferable range is 0.030 or more and 0.090 or less.

The upper limit of h is 4.0, 3.0, 2.0, 1.8, 1.5, 1.0, 0.80, or 0.50 in order of preference, and the lower limit thereof is preferably 0. That is, a more preferable range of h is 0 or more and 0.50 or less, and 0 is most preferable.

X in the formula (1) is preferably tungsten, antimony, zinc, magnesium, calcium, or cerium, and particularly preferably antimony or zinc.

Y in the formula (1) is preferably sodium, potassium, or cesium, and further preferably potassium or cesium.

Z in the formula (1) is preferably vanadium, copper, niobium, zirconium, calcium, beryllium, strontium, barium, lead, or phosphorus.

[First Layer Catalyst]

In the invention, the composition of the active components of the catalyst contained in the first layer (the catalyst layer closest to the inlet of the raw material gas for reaction) is preferably a catalyst represented by the following formula (1-1). In the case of filling with three or more layers, a catalyst having a composition represented by the formula (1-1) is preferable except for the side closest to the outlet.

[Formula 1-1]

$$Mo_{a1}Bi_{b1}Ni_{c1}Co_{d1}Fe_{e1}X_{f1}Cs_{g1}Z_{h1}O_{i1} \qquad (1\text{-}1)$$

In the formula (1-1), Mo, Bi, Ni, Co, Fe, and Cs respectively represent molybdenum, bismuth, nickel, cobalt, iron, and cesium, and X is at least one element selected from tungsten, antimony, tin, zinc, chromium, manganese, magnesium, silicon, aluminum, cerium, and titanium. Z belongs to Group 1 to Group 16 in the periodic table and means at least one element selected from elements other than the above Mo, Bi, Ni, Co, Fe, Cs, and X, and a1, b1, c1, d1, e1, f1, g1, h1, and i1 respectively represent the numbers of atoms of molybdenum, bismuth, nickel, cobalt, iron, X, Cs, Z, and oxygen. When $a1 = 12$, $0 < b1 \leq 7.0$, $0 \leq c1 \leq 10$, $0 < d1 \leq 10$, $0 < c1+d1 \leq 20$, $0 < e1 \leq 5.0$, $0 \leq f1 \leq 2.0$, $0 < g1 \leq 3.0$, and $0 \leq h1 \leq 5.0$ are satisfied, and i1 is a value determined by the oxidation state of each element.

Preferable b1 to f1 and h1 and X and Z when a1 = 12 in the formula (1-1) are the same as b to h and X and Z in the formula (1) including preferable embodiments.

The lower limit of g1 is 0.0010, 0.0050, 0.010, 0.015, 0.020, or 0.030 in order of preference, and the upper limit thereof is 2.0, 1.0, 0.50, 0.40, 0.30, 0.20, 0.15, 0.090, or 0.060 in order of preference. That is, g1 is preferably 0.0010 or more and 2.0 or less, more preferably 0.0010 or more and 1.0 or less, more preferably 0.0010 or more and 0.50 or less, more preferably 0.0010 or more and 0.40 or less, more preferably 0.0050 or more and 0.30 or less, more preferably 0.010 or more and 0.20 or less, more preferably 0.015 or more and 0.15 or less, and more preferably 0.020 or more and 0.090 or less, and the most preferable range is 0.030 or more and 0.060 or less.

[Catalyst Closest to Outlet]

In the invention, the composition of the active components of the catalyst contained in the side closest to the outlet (the catalyst layer closest to the outlet of the raw material gas for reaction) is preferably a catalyst represented by the following formula (1-2). For example, in the case of three-layer filling, it is preferable that the third layer is also a catalyst represented by the formula (1-2), and in the case of further multi-layer filling, it is preferable that the side closest to the outlet is the present catalyst.

[Formula 1-2]

$$Mo_{a2}Bi_{b2}Ni_{c2}Co_{d2}Fe_{e2}X_{f2}K_{g2}Z_{h2}O_{i2} \qquad (1\text{-}2)$$

In the formula, Mo, Bi, Ni, Co, Fe, and K respectively represent molybdenum, bismuth, nickel, cobalt, iron, and potassium, and X is at least one element selected from tungsten, antimony, tin, zinc, chromium, manganese, magnesium, silicon, aluminum, cerium, and titanium. Z belongs to Group 1 to Group 16 in the periodic table and means at least one element selected from elements other than the above Mo, Bi, Ni, Co, Fe, K, and X, and a2, b2, c2, d2, e2, f2, g2, h2, and i2

respectively represent the numbers of atoms of molybdenum, bismuth, nickel, cobalt, iron, X, K, Z, and oxygen. When $a2 = 12$, $0 < b2 \leq 7.0$, $0 \leq c2 \leq 10$, $0 < d2 \leq 10$, $0 < c2 + d2 \leq 20$, $0 < e2 \leq 5.0$, $0 \leq f2 \leq 2.0$, $0 \leq g2 \leq 3.0$, and $0 \leq h2 \leq 5.0$, and i2 is a value determined by the oxidation state of each element.

Preferable b2 to f1 and h2 and X and Z when a2 = 12 in the formula (1-2) are the same as b to h and X and Z in the formula (1) including preferable embodiments.

The lower limit of g2 is 0.0010, 0.0050, 0.010, 0.015, 0.020, 0.030, 0.040, or 0.050 in order of preference, and the upper limit thereof is 2.0, 1.0, 0.50, 0.40, 0.30, 0.20, 0.15, or 0.10 in order of preference. That is, g2 is preferably 0.0010 or more and 2.0 or less, more preferably 0.0010 or more and 1.0 or less, more preferably 0.0050 or more and 0.50 or less, more preferably 0.010 or more and 0.40 or less, more preferably 0.020 or more and 0.30 or less, more preferably 0.030 or more and 0.20 or less, and more preferably 0.040 or more and 0.15 or less, and the most preferable range is 0.050 or more and 0.10 or less.

The shapes of the catalysts contained in the catalyst layers used in the invention of the present application are not particularly restricted, and a spherical shape, a cylindrical shape, a ring shape, a powder shape, or the like can be used, but a spherical shape is preferable.

In the case of two-layer filling, the catalyst contained in the upper layer and the catalyst contained in the lower layer may be both diluted with an inert substance, but a method of diluting neither the upper layer nor the lower layer is more preferable.

Preferable ranges of the dilution rate di with an inert substance are shown below. The dilution rate here is a numerical value indicating the mass percentage that the catalyst accounts for in a catalyst layer composed of the catalyst and an inert substance, and for example, the dilution of the catalyst layer of 80 mass% means 80 mass% of the catalyst and 20 mass% of the inert substance. Here, as explained for the method for producing a catalyst described below, in the case where a catalytically active component is carried on an inert carrier to form a catalyst, the dilution rate is calculated based on the mass of the catalyst including the inert carrier. Hereinafter, a preferable form will be described using an example of the case where n = 2 and 3, but the invention of the present application is not limited thereto, since the nature thereof is that the fill length of the other catalyst layers with respect to the fill length of the n-th catalyst layer of the catalysts obtained by dividing the reaction tube into n in the gas flow direction is within a certain range.

Preferable embodiments in the invention of the present application are the following 1) and 2).

1) n = 2. The upper layer is a catalyst containing a catalytically active component represented by the formula (1-1) and has a dilution rate of 100 mass%, and the lower layer is a catalyst containing a catalytically active component represented by the formula (1-2) and has a dilution rate of 100 mass%.

2) n=3. The middle layer is a catalyst containing a catalytically active component represented by the formula (1-1) and has a dilution rate of 100 mass%, and the upper layer is a catalyst obtained by diluting the middle layer catalyst with an inert substance. The lower layer is a catalyst containing a catalytically active component represented by the formula (1-2) and has a dilution rate of 100 mass%.

There is also a filling method in which an inert substance layer is provided closer to the inlet than the catalyst layers in the reaction tube. As long as the effects of the invention are not inhibited, the filling method may be employed, but a method without the inert substance layer is preferable.

Examples of the inert substance include known substances such as silica, alumina, titania, zirconia, niobia, silica alumina, silicon carbide, carbide, magnesia, and mixtures thereof. Of these, silica, alumina, or a mixture thereof is preferable, and silica and alumina are particularly preferable. A mixture of silica and alumina is most preferable.

Moreover, the shape of the inert substance is not particularly restricted, and is preferably a spherical shape, and the average particle diameter thereof is preferably 3 mm to 10 mm, and further preferably 3.5 mm to 9 mm. The average particle diameter is particularly preferably 4 mm to 8 mm.

[Method for Producing Catalyst]

The catalysts used in the invention of the present application can be produced, for example, by the following steps a) to e).

<Step a) Preparation>

In general, the starting raw material of each element constituting the catalytically active component is not particularly restricted. As the molybdenum component raw material, molybdenum oxides such as molybdenum trioxide, molybdic acid or salts thereof such as molybdic acid and ammonium molybdate, molybdenum-containing heteropoly acids such as phosphomolybdic acid and silicomolybdic acid, or salts thereof, and the like can be used. Ammonium molybdate is preferably used, and a high-performance catalyst can be obtained. In particular, examples of ammonium molybdate

include a plurality of kinds of compounds such as ammonium dimolybdate, ammonium tetramolybdate, and ammonium heptamolybdate, and of these, ammonium heptamolybdate is most preferably used.

As the bismuth component raw material, bismuth salts such as bismuth nitrate, bismuth subcarbonate, bismuth sulfate, and bismuth acetate, bismuth trioxide, metal bismuth, or the like can be used, and bismuth nitrate is more preferable, and a high-performance catalyst is obtained when bismuth nitrate is used. As raw materials for iron, cobalt, nickel, and other elements, typically, an oxide, or a nitrate, a carbonate, an organic acid salt, a hydroxide, or the like which can be converted into an oxide by high heat, or mixtures thereof can be used. For example, an iron component raw material and a cobalt component raw material and/or a nickel component raw material are dissolved and mixed in water at a desired ratio under a condition of 10 to 80°C, and the mixture is mixed with a separately prepared aqueous solution or slurry of a molybdenum component raw material and a Z component raw material under a condition of 20 to 90°C. After the mixture is heated and stirred under a condition of 20 to 90°C for about one hour, an aqueous solution in which a bismuth component raw material is dissolved and, if necessary, an X component raw material and a Y component raw material, are added to obtain an aqueous solution or slurry containing the catalyst components. Hereinafter, both are collectively referred to as a prepared liquid (A).

Here, the prepared liquid (A) does not always necessarily contain all the elements constituting the catalytically active component, and a part of the elements or a part of the amount thereof may be added in a subsequent step. In addition, when preparing the prepared liquid (A), unless an amount of water for dissolving the component raw materials or the acid concentration in the aqueous solution sufficient to dissolve the raw materials in the case of adding an acid such as sulfuric acid, nitric acid, hydrochloric acid, tartaric acid, and acetic acid for dissolution, is suitable for preparation, for example, in the range of 5 mass% to 99 mass%, the prepared liquid (A) sometimes becomes in the form of a clay-like lump. In this case, an excellent catalyst cannot be obtained. The form of the prepared liquid (A) is preferably an aqueous solution or slurry as an excellent catalyst can be obtained.

<Step b) Drying>

Next, the prepared liquid (A) obtained above is dried to form a dry powder. The drying method is not limited as long as it is a method capable of completely drying the prepared liquid (A), and examples thereof include drum drying, freeze drying, spray drying, and evaporation to dryness. Among these, in the invention, spray drying is particularly preferred since the slurry can be dried into a powder or granules in a short period of time. The drying temperature in the spray drying varies depending on a concentration of the slurry, a liquid feeding rate, or the like, and the temperature at the outlet of a dryer is typically 70°C to 150°C. In addition, drying is preferably performed such that the average particle diameter of the dry powder obtained at this time is 10 to 700 μm. In this way, a dry powder (B) is obtained.

<Step c) Preliminary Calcination>

When the obtained dry powder (B) is calcined under air flow at 200°C to 600°C, preferably at 300°C to 600°C, the formability, the mechanical strength, and the catalyst performance of the catalyst tend to improve. The calcination time is preferably 1 hour to 12 hours. In this way, a preliminarily calcined powder (C) is obtained.

<Step d) Forming>

The forming method is not particularly limited, and in the case of forming into a cylindrical shape or a ring shape, a method using a tablet forming machine, an extrusion forming machine, or the like is preferred. A case of forming into a spherical shape is further preferable, and the preliminarily calcined powder (C) may be formed into a spherical shape using a forming machine, but a method of carrying the preliminarily calcined powder (C) (containing a forming aid and a strength improver if necessary) on a carrier such as inert ceramic is preferable. Here, as a carrying method, a tumbling granulation method, a method using a centrifugal fluid coating apparatus, a wash coating method, and the like are widely known. The method is not particularly limited as long as the preliminarily calcined powder (C) can be uniformly carried on the carrier. In consideration of the production efficiency of the catalyst and the performance of the prepared catalyst, more preferable is a method of rotating a flat or uneven disc at a high speed in an apparatus that has the disc at the bottom of a fixed columnar container, to vigorously agitate a carrier charged in the container by repeated rotation and revolution movements of the carrier itself, and adding, to the container, the preliminarily calcined powder (C), and if necessary a forming aid and/or a strength improver to carry the powder component on the carrier.

A binder is preferably used for carrying. Specific examples of the binder to be used include water, ethanol, methanol, propanol, a polyhydric alcohol, a polyvinyl alcohol that is a polymer-based binder, and a silica sol aqueous solution that is an inorganic binder. Ethanol, methanol, propanol, and a polyhydric alcohol are preferred, a diol such as ethylene glycol and a triol such as glycerin are more preferred. When an appropriate amount of an aqueous glycerin solution is used, the formability becomes excellent, and a high-performance catalyst having high mechanical strength can

be obtained. Specifically, a particularly high-performance catalyst can be obtained when an aqueous solution of glycerin having a concentration of 5 mass% or more is used. The amount of these binders to be used is typically 2 to 80 parts by mass with respect to 100 parts by mass of the preliminarily calcined powder (C). An inert carrier having a diameter of about 2 to 8 mm is typically used, and the preliminarily calcined powder (C) is carried thereon. The carrying ratio is determined in consideration of the catalyst usage conditions, for example, reaction conditions such as the space velocity of the reaction raw materials and the concentrations of the raw materials, and is typically 20 mass% to 80 mass%. Here, the carrying ratio is represented by the following equation.

Carrying ratio (mass%) =

100 × [mass of preliminarily calcined powder (C) used for forming/(mass of preliminarily calcined powder (C) used for forming + mass of inert carrier used for forming)] (2)

<Step e) Main Calcination>

When the formed body (D) obtained by the step d) is calcined at a temperature of 200 to 600°C for about one to 12 hours, the catalytic activity and the selectivity tend to improve. The calcination temperature is preferably 400°C or higher and 600°C or lower, and more preferably 500°C or higher and 600°C or lower. Air is convenient and preferred as a gas to be circulated. In addition, nitrogen, carbon dioxide, a nitrogen oxide-containing gas for creating a reducing atmosphere, an ammonia-containing gas, a hydrogen gas, and mixtures thereof can be also used as inert gases. In this way, a catalyst (E) is obtained.

[Method for Producing Catalyst Which Gives Preferable CB]

In order to obtain a catalyst having a production parameter T which satisfies the formula (I), it is generally preferable that the CB is low. Examples of the method for producing a catalyst having low CB include the following production methods. These can also be used singly or in combination.

(1) A first method is a method in which the pH of the prepared liquid is adjusted in a range of 1.0 to 7.5, and more preferably in a range of 3.0 to 6.0, before charging an iron raw material, in the preparation step of adding and integrating supply source compounds containing molybdenum, bismuth, and iron to a solvent or a solution and heating the mixture to prepare a prepared liquid. Examples of the adjustment of pH include a method of lowering the pH by adding nitric acid or the like, or a method of raising the pH by adding ammonia water or the like, which will be described in detail later.

As for the pH adjusting agent, in the method of lowering the pH, an acid for general pH adjustment that those skilled in the art use as a catalyst raw material, such as nitric acid, sulfuric acid, hydrochloric acid, and oxalic acid, can be used. In addition, a pH adjusting agent from which an element remains after calcination, such as phosphoric acid, boric acid, molybdic acid, and iron nitrate, can be used as long as the element composition of the catalytically active component to be described later is not changed, but nitric acid is most preferable. In the method of raising the pH, a base for general pH adjustment that those skilled in the art use as a catalyst raw material, such as ammonia water, pyridine, and an aqueous ammonium carbonate solution, can be used. In addition, a pH adjusting agent from which an element remains after calcination, such as potassium hydroxide and cesium hydroxide, can be used as long as the element composition of the catalytically active component to be described later is not changed, but ammonia water is most preferable.

In the case of performing this method, the most preferred embodiment is a method of preparing a slurry in the order of adding the raw material for iron component and then adding a bismuth raw material to the blended solution after stirring.

For dropwise adding of the pH adjusting agent, for example, the pH adjusting agent is charged while stirring at a stirring power of 0.01 to 5.00 kw/m$^3$. A lower limit of the stirring power is preferably 0.05 kw/m$^3$, 0.10 kw/m$^3$, 0.50 kw/m$^3$, and 1.00 kw/m$^3$, and an upper limit of the stirring power is preferably 4.50 kw/m$^3$, 4.00 kw/m$^3$, 3.50 kw/m$^3$, and 3.00 kw/m$^3$. That is, the most preferred range of the stirring power is 1.00 kw/m$^3$ to 3.00 kw/m$^3$.

The dropping time for the pH adjusting agent is 1 second to 5 minutes. The lower limit of the dropping time is preferably 5 seconds, 10 seconds, and 15 seconds, and the upper limit of the dropping time is preferably 4 minutes, 3 minutes, 2 minutes and 30 seconds, 2 minutes, 1 minute and 30 seconds, 1 minute, 45 seconds, and 30 seconds. That is, the most preferred range of the dropping time is 15 seconds to 30 seconds.

The liquid temperature of the prepared liquid at the time of dropwise addition of the pH adjusting agent is 5 to 80°C. The lower limit of the liquid temperature is preferably 10°C, 20°C, or 30°C, and the upper limit of the liquid temperature is preferably 70°C, 60°C, 50°C, or 40°C. That is, the most preferable range of the liquid temperature is 30 to

40°C.

(2) A second method is the optimization of the rotational speed of the sprayer (atomizer) in the case of spray drying. The optimum rotational speed of the atomizer is not unconditionally determined because the rotational speed is affected by the structure of the atomizer or the spray dryer, the temperature, the pH, and the viscosity of the liquid to be dried, the blending ratio of the catalyst components, and the like, but the rotational speed is preferably 10,000 rpm or more and 18,000 rpm or less. A further preferable upper limit of the rotational speed of the atomizer is 17,000 rpm, particularly preferably 16,000 rpm, and most preferably 15,000 rpm. A further preferred lower limit thereof is 11,000 rpm, a particularly preferred lower limit thereof is 12,000 rpm, and a most preferred lower limit thereof is 13,000 rpm. That is, the most preferable range of the rotational speed of the atomizer is 13,000 rpm or more and 15,000 rpm or less.

The rotational speed of the atomizer is also represented by relative centrifugal acceleration, and is preferably 6,000 G to 20,000G. A more preferred lower limit thereof is 7,500 G, 8,500 G, and 10,000 G in order, and a more preferred upper limit thereof is 18,000 G, 16,000 G, and 14,000 G in order. That is, the most preferable range of the rotational speed of the atomizer is 10000 G or more and 14000 G or less.

Furthermore, the hot air temperature supplied to the sprayer for spray drying (hereinafter sometimes referred to as the inlet temperature) and the spray dryer outlet temperature (hereinafter sometimes referred to as the outlet temperature) also affect the above parameters (CB and the production parameter T). An inlet temperature is preferably 180°C to 320°C. A more preferred lower limit thereof is 200°C, 220°C, and 230°C in order, and a more preferred upper limit thereof is 300°C, 280°C, and 270°C in order. That is, the inlet temperature is most preferably 230°C or higher and 270°C or lower.

Moreover, the outlet temperature is preferably 100°C or higher and 150°C or lower. A more preferred lower limit thereof is 101°C, 102°C, 103°C, 104°C, and 105°C in order, and a more preferred upper limit thereof is 140°C, 130°C, and 120°C in order. That is, the inlet temperature is most preferably 105°C or higher and 120°C or lower.

The difference between the inlet temperature and the outlet temperature is preferably 30°C or more and 220°C or less. A more preferred lower limit thereof is 50°C, 80°C, 100°C, and 120°C in order, and a more preferred upper limit thereof is 200°C, 180°C, 165°C, and 150°C in order. That is, the difference between the inlet temperature and the outlet temperature is most preferably 120°C or more and 150°C or less.

Further, the range of a parameter SD given by the following equation in the method for producing a catalyst precursor of the invention is preferably 22 or more and 51 or less. A more preferred lower limit thereof is 26, 28, 30, 32, 34, and 35 in order, and a more preferred upper limit thereof is 48, 44, 42, and 40 in order. That is, SD is most preferably 35 or more and 40 or less.

SD = 51.3 + 0.0766 × {(inlet temperature of spray dryer, unit: °C) - (outlet temperature of spray dryer, unit: °C)} - 0.00173 × (rotational speed of atomizer, unit: rpm)

(3) A third method is a method of controlling the composition of the active components of the catalyst in more detail, and specifically, the control is performed by one or a combination of the following defined composition ratios. That is, in the compositional formula (1) described above, the upper limit of e/b is preferably 2.90, and more preferably 2.80, and the lower limit of e/b is 0.10, 0.50, 1.00, 1.40, 1.50, or 1.90 in order of desirability. Therefore, e/b is preferably 0.10 or more and 2.90 or less, more preferably 0.50 or more and 2.90 or less, more preferably 1.00 or more and 2.90 or less, more preferably 1.40 or more and 2.90 or less, more preferably 1.50 or more and 2.90 or less, and particularly preferably 1.90 or more and 2.80 or less.

The upper limit of d/b is 9.0, 8.0, or 7.0 in order of desirability, and the lower limit of d/b is 2.0, 3.0, 4.0, 5.0, 5.5, or 6.5 in order of desirability. Therefore, d/b is preferably 2.0 or more and 9.0 or less, more preferably 3.0 or more and 9.0 or less, more preferably 4.0 or more and 9.0 or less, more preferably 5.0 or more and 9.0 or less, more preferably 5.5 or more and 8.0 or less, and particularly preferably 6.5 or more and 7.0 or less.

The upper limit of c/e is 4.0, 3.0, 2.5, or 2.0 in order of desirability, and the lower limit of c/e is 1.0, 1.5, 1.7, or 1.9 in order of desirability. Therefore, c/e is preferably 1.0 or more and 4.0 or less, more preferably 1.5 or more and 3.0 or less, more preferably 1.7 or more and 2.5 or less, and particularly preferably 1.9 or more and 2.0 or less.

The upper limit of c/d is 2.0, 1.0, or 0.8 in order of desirability, and the lower limit of c/d is 0.1 or 0.3 in order of desirability. Therefore, c/d is preferably 0.1 or more and 2.0 or less, more preferably 0.1 or more and 1.0 or less, and particularly preferably 0.3 or more and 0.8 or less.

The upper limit of g/d is 0.100, 0.050, 0.040, 0.030, 0.020, or 0.015 in order of desirability, and the lower limit of g/d is 0.007, 0.008, 0.009, 0.010, or 0.011 in order of preference. Therefore, g/d is preferably 0.007 or more and 0.100 or less, more preferably 0.007 or more and 0.050 or less, more preferably 0.008 or more and 0.040 or less, more preferably 0.009 or more and 0.030 or less, more preferably 0.010 or more and 0.020 or less, and particularly preferably 0.011 or more and 0.015 or less.

The upper limit of g/c is 0.040, 0.035, or 0.030 in order of desirability, and the lower limit of g/c is 0.015, 0.020, or

0.025 in order of desirability. Therefore, g/c is preferably 0.015 or more and 0.040 or less, more preferably 0.020 or more and 0.035 or less, and particularly preferably 0.025 or more and 0.030 or less.

(4) A fourth method is control of the calcination temperature, the calcination time, or the calcination atmosphere in the preliminary calcination or the main calcination described below or both thereof. The calcination temperature is set to 200°C or higher and 600°C or lower, preferably 300°C or higher and 550°C or lower, and more preferably 460°C or higher and 550°C or lower. The calcination time is set to 0.5 hours or longer, preferably one hour or longer and 40 hours or shorter, more preferably two hours or longer and 15 hours or shorter, and most preferably two hours or longer and nine hours or shorter. The calcination atmosphere is at an oxygen concentration of 10 vol% or more and 40 vol% or less, and preferably 15 vol% or more and 30 vol% or less and is most preferably an air atmosphere.

(5) In a fifth method, in the preliminary calcination or the main calcination described below or both thereof, the rate of decrease in the temperature of the catalyst surface (temperature-lowering rate) from the maximum temperature reached during the calcination step (the preliminary calcination temperature or the main calcination temperature) to room temperature is controlled. That is, the temperature-lowering rate is set to 1°C/min or more and 200°C/min or less, preferably 5°C/min or more and 150°C/min or less, more preferably 10°C/min or more and 120°C/min or less, most preferably 50°C/min or more and 100°C/min or less. Temperature lowering methods to be typically used industrially in order to implement the above temperature-lowering rate range, for example, a method of exposing a catalyst taken out from a firing furnace after calcination to an inert atmosphere or mist of an inert solvent, and a method of rapidly moving a catalyst after calcination into a sufficiently pre-cooled chamber, are all within the scope of the invention.

(6) A sixth method is a method of controlling the catalyst precursor which will be described later and/or the granules formed in each step in such a manner that mechanical impact, shear stress, and the like are not applied. The mechanical impact, the shear stress, and the like are controlled to preferably 100 kgf or less, more preferably 50 kgf or less, more preferably 20 kgf or less, further preferably 10 kgf or less, and most preferably 5 kgf or less.

(7) A seventh method is a method using a reagent-grade high-purity raw material. Although the details are not limited, for example, as the raw material, a raw material in which the content of sulfur and a compound thereof, lithium, a halogen and a compound thereof, and lead is 10000 ppm by weight or less, preferably 1000 ppm by weight or less, more preferably 100 ppm by weight, and most preferably 10 ppm by weight or less is used.

(8) An eighth method is a method of controlling the time for which the cobalt raw material and the nickel raw material are mixed with other raw materials in a preparation vessel and/or the time for reacting on each other and/or the time for slurrying and/or the time for retention in the preparation step of the catalyst which will be described later so as to be as short as possible. A more specific method is a method of shortening the retention time in a state where no metal salt raw materials other than molybdenum and alkali metals are in the preparation vessel but there are the cobalt raw material and the nickel raw material, or a method of shortening the retention time in a state where there are the cobalt raw material and the nickel raw material when the pH in the preparation vessel is in a specific range. The retention time is preferably 24 hours or less, further preferably one hour or less, further preferably 30 minutes or less, and most preferably 10 minutes or less. The pH range of the solution in the preparation vessel is preferably 2 or more and 10 or less, more preferably 2 or more and 8 or less, and most preferably 3 or more and 7 or less. The same applies to the iron raw material and the bismuth raw material, and the molybdenum raw material and the bismuth raw material.

(9) A ninth method is a method of charging each raw materials at once in the preparation step undividedly or a method of reducing the nitric acid concentration of the prepared liquid, in the preparation step of the catalyst which will be described later. The above method of charging at once means charging a next raw material after all the required amount of each raw materials is charged. Moreover, regarding the nitric acid concentration of the prepared liquid, the nitrate ion concentration is adjusted to preferably 40 mass% or less, more preferably 35 mass% or less, further preferably 30 mass% or less, and most preferably 25 mass% or less, in the prepared liquid at the time of proceeding to the next step after the completion of the preparation.

[Concentration of Alkene in Raw Material]

The catalytic gas-phase oxidation reaction of the alkene in the invention is carried out by introducing a mixed gas containing 6 to 12 vol% of alkene (more preferably 6 to 10 vol%), 5 to 18 vol% of molecular oxygen, 0 to 60 vol% of water vapor, 20 to 70 vol% of an inert gas such as nitrogen or carbon dioxide gas, and the like, as a raw material gas composition, onto a catalyst prepared as described above in a temperature range of 250 to 450°C and under a pressure of atmospheric pressure to 10 atm, preferably under atmospheric pressure to 5 atm, and more preferably under atmospheric pressure to 3 atm, for a contact time of 0.5 to 10 seconds.

Moreover, it is particularly preferable that the volume ratio of the alkene and the oxygen (oxygen/alkene) is 1.0 or more and 1.9 or less. A more preferable upper limit of oxygen/alkene is 1.8, 1.7, or 1.6 in this order, and the upper limit is more preferably 1.5. Moreover, a more preferable lower limit is 1.1, 1.2, or 1.3 in this order. From the above, the most preferable range of oxygen/alkene is 1.3 or more and 1.5 or less.

In the present invention, the term "alkene" includes alcohols that generate alkenes by an intramolecular

dehydration reaction thereof, for example, tertiary butyl alcohol. The space velocity with respect to the catalyst volume of the reaction substrate (raw material) such as the alkene (SVo = reaction substrate feed rate (NL/hr)/catalyst-filled space volume (L)) is preferably high from the viewpoint of production efficiency, but, in practice, is preferably 40 to 200 $hr^{-1}$, and more preferably in the range of 60 to 180 $hr^{-1}$ because the yield of the target product may be lowered or the lifetime of the catalyst is shortened when the space velocity is too high. Here, NL represents the volume of the reaction substrate in the standard state (1 atm, 0°C). Moreover, the conversion rate of the alkene is preferably in the vicinity of the conversion rate at which a high unsaturated aldehyde yield is obtained and is typically 90 to 99.9%, preferably 95 to 99.5%, and more preferably 96 to 99%.

When the catalyst layer on the reaction gas outlet side is made too short to obtain the effects of the invention, the reaction bath temperature required to obtain the usual raw material conversion rate and to obtain the target product is excessively increased because the activity of the entire catalyst layers decreases. Thus, the temperature of the hot spots becomes high, and degradation and performance deterioration of the catalyst occur. Moreover, in some cases, early degradation of the catalyst on the gas inlet side may cause high hot spots in the highly active catalyst layer on the gas outlet side, causing a sharp drop in the selectivity and the yield of the target product. Therefore, in consideration of the balance between the catalyst on the gas inlet side and the catalyst on the gas outlet side, it is also necessary to prevent the activity of the entire catalyst layers from lowering and the reaction bath temperature from excessively increasing, which are otherwise caused when the catalyst layer on the outlet side is too short. The reaction bath temperature is not unconditionally determined because the reaction bath temperature is appropriately set depending on the characteristics of the catalyst, the usage conditions, the required catalyst life, and the like, and the reaction bath temperature at the initial stage of the reaction is preferably 390°C or lower. The upper limit thereof is 380, 370, 360, or 350 in order of preference, and the upper limit is most preferably 340°C or lower. Moreover, the lower limit thereof is 300°C or higher, and more preferably 310°C or higher. Here, the reaction bath temperature means the set temperature for achieving an appropriate raw material conversion rate, not the temperature in the temperature raising process.

Regarding the control of the fill length of the invention, L/Ln is preferably 0.1 or more and 1.0 or less, when the sum of the fill lengths of the first to n-1-th catalyst layers counted from the reaction gas inlet side is L, and the fill length of the n-th catalyst layer counted from the reaction gas inlet side is Ln. A preferable lower limit of L/Ln is 0.2, 0.3, or 0.4 in order of preference, and a preferable upper limit thereof is 0.9. Therefore, L/Ln is more preferably 0.2 or more and 1.0 or less, more preferably 0.3 or more and 1.0 or less, and particularly preferably 0.4 or more and to 0.9 or less.

In an industrial plant, by carrying out the production method above, the yield of the unsaturated aldehyde can be improved, runaway of the highly active gas outlet side can be suppressed, and stable yield and operation become possible in the industrial plant for a long period of time. This effect is believed to be caused by an increase in the contribution of the catalyst having a high selectivity because the occupancy ratio of the catalyst layer having a relatively high selectivity exceeds the occupancy ratio of the catalyst layer having a relatively high activity.

Example

Although Examples are shown below by giving specific examples, the invention is not limited to the Examples unless it deviates from the spirit thereof.

In the following, the definition of the effective yield is as follows.

Propylene conversion rate (mol%)

= (number of moles of reacted propylene/number of moles of propylene supplied) × 100

Effective yield (mol%)

= (number of moles of acrolein and acrylic acid produced/number of moles of propylene supplied) × 100

Effective selectivity (mol%)

= (number of moles of acrolein and acrylic acid produced/number of moles of propylene reacted) × 100

[Production Example (Preparation of Catalyst)]

(Catalyst 1)

A catalyst 1 was produced by the method shown below in such a manner that the composition ratio of the catalytically active components excluding oxygen became Mo:Bi:Fe:Co:Ni:Cs = 12:0.7:2.0:6.2:2.0:0.04 in atomic ratio. An

aqueous solution (prepared liquid 1) was obtained by dissolving ammonium molybdate and cesium nitrate in distilled water while heating and stirring. Here, the mass of the distilled water used was 3.5 times the mass of the ammonium molybdate, and the water temperature at the time of charging the ammonium molybdate was 85°C. Separately, an aqueous solution (prepared liquid 2) was prepared by dissolving cobalt nitrate, nickel nitrate, and ferric nitrate in distilled water, and an aqueous solution (prepared liquid 3) was prepared by dissolving bismuth nitrate in distilled water acidified by adding concentrated nitric acid. After concentrated nitric acid was added to the prepared liquid 1, the prepared liquid 2 and the prepared liquid 3 were sequentially mixed with vigorous stirring. The resulting suspension was dried using a spray dryer, and the obtained granule was calcined at 440°C for six hours to obtain a preliminarily calcined powder. Thereafter, the carrier mass and the preliminarily calcined powder mass used for forming were adjusted in such a manner that the carrying ratio defined by the above equation became 60 mass% in a powder obtained by mixing crystalline cellulose in the preliminarily calcined powder on an inert carrier (a spherical substance containing alumina and silica as main components and having a diameter of 4.0 mm). A 33 mass% glycerin aqueous solution was used as a binder, and a carried catalyst was obtained by carrying and forming into spheres having a diameter of 5.1 mm. The carried catalyst was calcined at a calcination temperature of 520°C for four hours in an air atmosphere to obtain the catalyst 1.

A reaction tube having an inner diameter of 28.4 mm was filled with 4 g of the catalyst 1 in a state of being diluted with an inert substance so as not to generate any hot spot, and reaction was carried out at a molar ratio of propylene:oxygen:nitrogen:water = 1:1.7:6.4:3.0 at a space velocity (SVo) of propylene set at 400 $hr^{-1}$ with changing the BT at a reaction bath temperature from 340°C to 360°C. Thus, the propylene conversion rates were determined. The results and the calculated CB are shown in Table 1-1.

[Table 1-1]

| BT [°C] | Propylene Conversion Rate [%] |
|---|---|
| 340 | 20.2 |
| 360 | 26.3 |
| CB | 0.305 |

(Catalyst 2)

A catalyst 2 having a diameter of 5.3 mm was obtained by producing exactly in the same manner as the catalyst 1 except that, in the production of the catalyst 1, the raw material was changed from cesium nitrate to potassium nitrate, the composition ratio was Mo:Bi:Fe:Co:Ni:K = 12:0.8:1.6:6.1:2.9:0.07, the diameter of the inert carrier was 4.5 mm, and the carrying ratio was 50 mass%. The results of evaluation in the same manner as for the catalyst 1 and the calculated CB are shown in Table 1-2.

[Table 1-2]

| BT [°C] | Propylene Conversion Rate [%] |
|---|---|
| 340 | 30.4 |
| 360 | 42.2 |
| CB | 0.590 |

(Catalyst 3)

A catalyst 3 having a diameter of 5.2 mm was obtained by producing exactly in the same manner as the catalyst 1 except that, in the production of the catalyst 1, concentrated nitric acid was not added to the prepared liquid 1 in the preparation step, the composition ratio was Mo:Bi:Fe:Co:Ni:Cs = 12:0.9:2.0:6.5:3.0:0.03, the diameter of the inert carrier was 4.5 mm, the carrying ratio was 50 mass%, and the calcination temperature was 540°. The results of evaluation in the same manner as for the catalyst 1 and the calculated CB are shown in Table 1-3.

[Table 1-3]

| BT [°C] | Propylene Conversion Rate [%] |
|---|---|
| 340 | 24.2 |
| 360 | 29.5 |

(continued)

| BT [°C] | Propylene Conversion Rate [%] |
|---|---|
| CB | 0.265 |

(Catalyst 4)

A catalyst 4 having a diameter of 5.0 mm was obtained by producing exactly in the same manner as the catalyst 1 except that, in the production of the catalyst 1, concentrated nitric acid was not added to the prepared liquid 1 in the preparation step, the raw material was changed from cesium nitrate to potassium nitrate, the composition ratio was Mo:Bi:Fe:Co:Ni:K = 12:0.9:1.7:6.0:3.3:0.06, and the carrying ratio was 60 mass%. The results of evaluation in the same manner as for the catalyst 1 and the calculated CB are shown in Table 1-4.

[Table 1-4]

| BT [°C] | Propylene Conversion Rate [%] |
|---|---|
| 340 | 32.7 |
| 360 | 40.1 |
| CB | 0.370 |

(Catalyst 5)

A catalyst 5 having a diameter of 5.2 mm was obtained by producing exactly in the same manner as the catalyst 2 except that, in the production of the catalyst 2, concentrated nitric acid was not added to the prepared liquid 1 in the preparation step, the composition ratio was Mo:Bi:Fe:Co:Ni:K = 12:0.9:2.0:6.5:3.0:0.05, the diameter of the inert carrier was 4.5 mm, the carrying ratio was 50 mass%, and the calcination temperature was 550°. The results of evaluation in the same manner as for the catalyst 1 and the calculated CB are shown in Table 1-5.

[Table 1-5]

| BT [°C] | Propylene Conversion Rate [%] |
|---|---|
| 340 | 18.9 |
| 360 | 46.9 |
| CB | 1.40 |

(Catalyst 6)

A catalyst 6 was obtained by producing exactly in the same manner as the catalyst 1 except that the calcination temperature was 510°C in the production of the catalyst 1. The results of evaluation in the same manner as for the catalyst 1 and the calculated CB are shown in Table 1-6.

[Table 1-6]

| BT [°C] | Propylene Conversion Rate [%] |
|---|---|
| 340 | 22.2 |
| 360 | 29.1 |
| CB | 0.345 |

(Catalyst 7)

A catalyst 7 was obtained by producing exactly in the same manner as the catalyst 2 except that the calcination temperature was 530°C in the production of the catalyst 2. The results of evaluation in the same manner as for the catalyst 1 and the calculated CB are shown in Table 1-7.

[Table 1-7]

| BT [°C] | Propylene Conversion Rate [%] |
|---|---|
| 340 | 32.4 |
| 360 | 44.2 |
| CB | 0.590 |

[Example 1]

In a stainless steel reactor having an inner diameter of 25 mm in which a jacket for circulating a melted salt as a heating medium and a thermocouple for measuring the temperatures of the catalyst layers were installed on the tube shaft, a thermowell for the thermocouple having an outer diameter of 3 mm was installed, and as shown in Table 2-1, a catalyst 1-diluted layer, the catalyst 1, and the catalyst 2 were packed in the order of the first layer to the third layer. The supplied amounts of propylene, air, water, and nitrogen were set in such a manner that the raw material molar ratio became propylene:oxygen (oxygen contained in the supplied air):water:nitrogen (nitrogen supplied separately from air) = 1:1.7:1.0:2.4. The supplied raw materials were allowed to flow in such a manner that the space velocity (SVo) of propylene became 90 $hr^{-1}$, and when 300 hours had passed after starting the reaction at a pressure at the outlet side of the reaction tube of 50 kPaG at the time of the total gas flow, the reaction bath temperature was changed, and the oxidation reaction of propylene was carried out. The reaction results were examined with changing the reaction bath temperature, and the results thereof are shown in Table 3-1. Here, the rows without any peak temperature or reaction result of the layer in A1 and A2 in Table 3-1 mean that A1 was calculated by linearly approximating from the data of BT (A°C) at which the effective yield was the maximum and a temperature lower than BTand that A2 was calculated by linearly approximating from the data of A and a temperature higher than A (the same applies hereinafter).

[Table 2-1]

| Catalyst Type | Dilution Rate d [%] | Fill Length [cm] | CB [%/°C] | S [%/°C] | Savg [%/°C] |
|---|---|---|---|---|---|
| Catalyst 1 | 75 | 65 | 0.305 | 0.0425 | 0.149 |
| Catalyst 1 | 100 | 95 | 0.305 | 0.0828 | |
| Catalyst 2 | 100 | 190 | 0.590 | 0.320 | |

[Table 3-1]

| BT [°C] | | Propylene Conversion Rate [%] | Effective Yield [%] | Effective Selectivity [%] |
|---|---|---|---|---|
| | 318 | 97.8 | 92.2 | 94.3 |
| | 322 | 98.4 | 92.5 | 94.0 |
| | 324 | 98.7 | 92.6 | 93.8 |
| A | 326 | 99.0 | 92.7 | 93.7 |
| | 333 | 99.3 | 92.5 | 93.1 |
| | 339 | 99.5 | 92.1 | 92.5 |
| | 342 | 99.6 | 91.9 | 92.3 |
| A1 | 310 | | 91.7 | |
| A2 | 346 | | 91.7 | |

[Example 2]

As shown in Table 2-2, a catalyst 3-diluted layer, the catalyst 3, and the catalyst 4 were packed in the order of the first layer to the third layer, and the oxidation reaction of propylene was carried out exactly under the same reaction conditions by the aging method as in Example 1. The reaction results were examined with changing the reaction bath temperature, and the results thereof are shown in Table 3-2.

[Table 2-2]

| Catalyst Type | Dilution Rate d [%] | Fill Length [cm] | CB [%/°C] | S [%/°C] | Savg [%/°C] |
|---|---|---|---|---|---|
| Catalyst 3 | 75 | 70 | 0.265 | 0.0398 | 0.103 |
| Catalyst 3 | 100 | 90 | 0.265 | 0.0681 | |
| Catalyst 4 | 100 | 190 | 0.370 | 0.201 | |

[Table 3-2]

| BT [°C] | | Propylene Conversion Rate [%] | Effective Yield [%] | Effective Selectivity [%] |
|---|---|---|---|---|
| | 315 | 96.8 | 91.3 | 94.3 |
| | 318 | 97.7 | 91.9 | 94.1 |
| | 321 | 98.2 | 92.2 | 93.9 |
| A | 324 | 98.5 | 92.3 | 93.7 |
| | 327 | 98.7 | 92.2 | 93.4 |
| | 333 | 99.1 | 92.1 | 92.9 |
| A1 | 315 | | 91.3 | |
| A2 | 370 | | 91.3 | |

[Example 3]

As shown in Table 2-3, a catalyst 3-diluted layer, the catalyst 3, and the catalyst 4 were packed in the order of the first layer to the third layer, and the oxidation reaction of propylene was carried out exactly under the same reaction conditions by the aging method as in Example 1 except that the raw materials were allowed to flow in such a manner that the space velocity (SVo) of propylene became 120 $hr^{-1}$. The reaction results were examined with changing the reaction bath temperature, and the results thereof are shown in Table 3-3.

[Table 2-3]

| Catalyst Type | Dilution Rate d [%] | Fill Length [cm] | CB [%/°C] | S [%/°C] | Savg [%/°C] |
|---|---|---|---|---|---|
| Catalyst 3 | 75 | 70 | 0.265 | 0.0398 | 0.103 |
| Catalyst 3 | 100 | 90 | 0.265 | 0.0681 | |
| Catalyst 4 | 100 | 190 | 0.370 | 0.201 | |

[Table 3-3]

| BT [°C] | | Propylene Conversion Rate [%] | Effective Yield [%] | Effective Selectivity [%] |
|---|---|---|---|---|
| | 320 | 97.1 | 90.9 | 93.6 |
| | 323 | 97.7 | 91.0 | 93.1 |
| A | 326 | 98.1 | 91.4 | 93.2 |
| | 329 | 98.4 | 91.2 | 92.7 |
| | 332 | 98.6 | 91.3 | 92.6 |
| A1 | 316 | | 90.4 | |
| A2 | 343 | | 90.4 | |

[Comparative Example 1]

As shown in Table 2-4, a catalyst 3-diluted layer, the catalyst 3, and the catalyst 5 were packed in the order of the first layer to the third layer, and the oxidation reaction of propylene was carried out exactly under the same reaction

conditions by the aging method as in Example 1. The reaction results were examined with changing the reaction bath temperature, and the results thereof are shown in Table 3-4.

[Table 2-4]

| Catalyst Type | Dilution Rate d [%] | Fill Length [cm] | CB [%/°C] | S [%/°C] | Savg [%/°C] |
|---|---|---|---|---|---|
| Catalyst 3 | 85 | 80 | 0.265 | 0.0515 | |
| Catalyst 3 | 100 | 80 | 0.265 | 0.0606 | 0.291 |
| Catalyst 5 | 100 | 190 | 1.40 | 0.760 | |

[Table 3-4]

| BT [°C] | | Propylene Conversion Rate [%] | Effective Yield [%] | Effective Selectivity [%] |
|---|---|---|---|---|
| | 315 | 93.6 | 88.2 | 94.2 |
| | 318 | 97.2 | 91.1 | 93.7 |
| A | 321 | 98.5 | 91.7 | 93.1 |
| | 323 | 98.8 | 91.7 | 92.8 |
| | 327 | 99.1 | 91.3 | 92.1 |
| | 331 | 99.4 | 91.4 | 92.0 |
| A1 | 319 | | 90.7 | |
| A2 | 340 | | 90.7 | |

[Example 4]

In a stainless steel reactor having an inner diameter of 25 mm in which a jacket for circulating a melted salt as a heating medium and a thermocouple for measuring the temperatures of the catalyst layers were installed on the tube shaft, a thermowell for the thermocouple having an outer diameter of 3 mm was installed, and as shown in Table 2-5, the catalyst 1 and the catalyst 2 were packed in the order of the first layer and the second layer. The supplied amounts of propylene, air, water, and nitrogen were set in such a manner that the raw material molar ratio became propylene:oxygen (oxygen contained in the supplied air):water:nitrogen (nitrogen supplied separately from air) = 1: 1.7:0.7:2.5. The supplied raw materials were allowed to flow in such a manner that the space velocity (SVo) of propylene became 140 $hr^{-1}$, and when 300 hours had passed after starting the reaction at a pressure at the outlet side of the reaction tube of 90 kPaG at the time of the total gas flow, the reaction bath temperature was changed, and the oxidation reaction of propylene was carried out. The reaction results were examined with changing the reaction bath temperature, and the results thereof are shown in Table 3-5.

[Table 2-5]

| Catalyst Type | Dilution Rate d [%] | Fill Length [cm] | CB [%/°C] | S [%/°C] | Savg [%/°C] |
|---|---|---|---|---|---|
| Catalyst 1 | 100 | 90 | 0.305 | 0.0915 | 0.252 |
| Catalyst 2 | 100 | 210 | 0.590 | 0.413 | |

[Table 3-5]

| BT [°C] | | Propylene Conversion Rate [%] | Effective Yield [%] | Effective Selectivity [%] |
|---|---|---|---|---|
| | 315 | 95.9 | 90.5 | 94.4 |
| | 326 | 98.2 | 92.2 | 93.9 |
| A | 329 | 98.5 | 92.4 | 93.8 |
| | 332 | 98.8 | 92.3 | 93.4 |
| | 336 | 98.8 | 92.1 | 93.2 |
| A1 | 320 | | 91.3 | |

(continued)

| BT [°C] | | Propylene Conversion Rate [%] | Effective Yield [%] | Effective Selectivity [%] |
|---|---|---|---|---|
| A2 | 355 | | 91.3 | |

[Example 5]

As shown in Table 2-6, the catalyst 3 and the catalyst 4 were packed in the order of the first layer and the second layer, and the oxidation reaction of propylene was carried out exactly under the same reaction conditions by the aging method as in Example 4. The reaction results were examined with changing the reaction bath temperature, and the results thereof are shown in Table 3-6.

[Table 2-6]

| Catalyst Type | Dilution Rate d [%] | Fill Length [cm] | CB [%/°C] | S [%/°C] | Savg [%/°C] |
|---|---|---|---|---|---|
| Catalyst 3 | 100 | 90 | 0.265 | 0.0795 | 0.169 |
| Catalyst 4 | 100 | 210 | 0.370 | 0.259 | |

[Table 3-6]

| BT [°C] | | Propylene Conversion Rate [%] | Effective Yield [%] | Effective Selectivity [%] |
|---|---|---|---|---|
| | 316 | 96.6 | 91.0 | 94.2 |
| | 319 | 97.1 | 91.5 | 94.2 |
| | 322 | 97.6 | 91.5 | 93.7 |
| A | 325 | 97.9 | 91.8 | 93.7 |
| | 328 | 98.1 | 91.8 | 93.6 |
| | 331 | 98.5 | 91.7 | 93.1 |
| A1 | 313 | | 90.8 | |
| A2 | 372 | | 90.8 | |

[Comparative Example 2]

As shown in Table 2-7, a catalyst 3-diluted layer, the catalyst 3, and the catalyst 5 were packed in the order of the first layer to the third layer, and the oxidation reaction of propylene was carried out exactly under the same reaction conditions by the aging method as in Example 4. The reaction results were examined with changing the reaction bath temperature, and the results thereof are shown in Table 3-7.

[Table 2-7]

| Catalyst Type | Dilution Rate d 1%] | Fill Length [cm] | CB [%/°C] | S [%/°C] | Savg [%/°C] |
|---|---|---|---|---|---|
| Catalyst 3 | 85 | 70 | 0.265 | 0.0509 | 0.293 |
| Catalyst 3 | 100 | 70 | 0.265 | 0.0598 | |
| Catalyst 5 | 100 | 170 | 1.40 | 0.768 | |

[Table 3-7]

| BT [°C] | | Propylene Conversion Rate [%] | Effective Yield [%] | Effective Selectivity [%] |
|---|---|---|---|---|
| | 318 | 96.7 | 90.3 | 93.4 |
| | 324 | 98.1 | 91.0 | 92.8 |
| A | 326 | 98.4 | 91.2 | 92.7 |
| | 328 | 98.5 | 91.0 | 92.4 |

(continued)

| BT [°C] | | Propylene Conversion Rate [%] | Effective Yield [%] | Effective Selectivity [%] |
|---|---|---|---|---|
| | 330 | 98.6 | 90.9 | 92.2 |
| A1 | 317 | | 90.2 | |
| A2 | 339 | | 90.2 | |

[Example 6]

In a stainless steel reactor having an inner diameter of 27 mm in which a jacket for circulating a melted salt as a heating medium and a thermocouple for measuring the temperatures of the catalyst layers were installed on the tube shaft, a thermowell for the thermocouple having an outer diameter of 3 mm was installed, and as shown in Table 2-8, a catalyst 3-diluted layer, the catalyst 3, and the catalyst 4 were packed in the order of the first layer to the third layer. The supplied amounts of propylene, air, water, and nitrogen were set in such a manner that the raw material molar ratio became propylene:oxygen (oxygen contained in the supplied air):water:nitrogen (nitrogen supplied separately from air) = 1:1.6:0.8:3.3. The supplied raw materials were allowed to flow in such a manner that the space velocity (SVo) of propylene became 84 $hr^{-1}$, and when 300 hours had passed after starting the reaction at a pressure at the outlet side of the reaction tube of 35 kPaG at the time of the total gas flow, the reaction bath temperature was changed, and the oxidation reaction of propylene was carried out. The reaction results were examined with changing the reaction bath temperature, and the results thereof are shown in Table 3-8.

[Table 2-8]

| Catalyst Type | Dilution Rate d [%] | Fill Length [cm] | CB [%/°C] | S [%/°C] | Savg [%/°C] |
|---|---|---|---|---|---|
| Catalyst 3 | 75 | 65 | 0.265 | 0.0391 | 0.103 |
| Catalyst 3 | 100 | 85 | 0.265 | 0.0683 | |
| Catalyst 4 | 100 | 180 | 0.370 | 0.202 | |

[Table 3-8]

| BT [°C] | | Propylene Conversion Rate [%] | Effective Yield [%] | Effective Selectivity [%] |
|---|---|---|---|---|
| | 310 | 87.2 | 82.4 | 94.5 |
| | 314 | 97.3 | 91.7 | 94.3 |
| A | 318 | 98.3 | 92.4 | 94.0 |
| | 320 | 98.6 | 92.4 | 93.7 |
| | 326 | 99.1 | 92.3 | 93.2 |
| A1 | 316 | | 91.4 | |
| A2 | 390 | | 91.4 | |

[Comparative Example 3]

As shown in Table 2-9, a catalyst 3-diluted layer, the catalyst 3, and the catalyst 5 were packed in the order of the first layer to the third layer, and the oxidation reaction of propylene was carried out exactly under the same reaction conditions by the aging method as in Example 6. The reaction results were examined with changing the reaction bath temperature, and the results thereof are shown in Table 3-9.

[Table 2-9]

| Catalyst Type | Dilution Rate d [%] | Fill Length [cm] | CB [%/°C] | S [%/°C] | Savg [%/°C] |
|---|---|---|---|---|---|
| Catalyst 3 | 85 | 80 | 0.265 | 0.0515 | 0.291 |
| Catalyst 3 | 100 | 80 | 0.265 | 0.0606 | |

(continued)

| Catalyst Type | Dilution Rate d [%] | Fill Length [cm] | CB [%/°C] | S [%/°C] | Savg [%/°C] |
|---|---|---|---|---|---|
| Catalyst 5 | 100 | 190 | 1.40 | 0.760 | |

[Table 3-9]

| BT [°C] | | Propylene Conversion Rate [%] | Effective Yield [%] | Effective Selectivity [%] |
|---|---|---|---|---|
| | 315 | 93.7 | 88.2 | 94.1 |
| | 317 | 97.4 | 91.2 | 93.6 |
| A | 318 | 98.6 | 91.5 | 92.8 |
| | 321 | 99.1 | 91.2 | 92.0 |
| | 324 | 99.3 | 91.0 | 91.7 |
| A1 | 317 | | 90.5 | |
| A2 | 330 | | 90.5 | |

[Example 7]

In a stainless steel reactor having an inner diameter of 28 mm in which a jacket for circulating a melted salt as a heating medium and a thermocouple for measuring the temperatures of the catalyst layers were installed on the tube shaft, a thermowell for the thermocouple having an outer diameter of 7 mm was installed, and as shown in Table 2-10, the catalyst 1 and the catalyst 7 were packed in the order of the first layer and the second layer. The supplied amounts of propylene, air, water, and nitrogen were set in such a manner that the raw material molar ratio became propylene:oxygen (oxygen contained in the supplied air):water:nitrogen (nitrogen supplied separately from air) = 1:1.7:1.2:3.0. The supplied raw materials were allowed to flow in such a manner that the space velocity (SVo) of propylene became 180 $hr^{-1}$, and when 300 hours had passed after starting the reaction at a pressure at the outlet side of the reaction tube of 80 kPaG at the time of the total gas flow, the reaction bath temperature was changed, and the oxidation reaction of propylene was carried out. The reaction results were examined with changing the reaction bath temperature, and the results thereof are shown in Table 3-10.

[Table 2-10]

| Catalyst Type | Dilution Rate d [%] | Fill Length [cm] | CB [%/°C] | S [%/°C] | Savg [%/°C] |
|---|---|---|---|---|---|
| Catalyst 1 | 100 | 90 | 0.305 | 0.0780 | 0.259 |
| Catalyst 7 | 100 | 262 | 0.590 | 0.439 | |

[Table 3-10]

| BT [°C] | | Propylene Conversion Rate [%] | Effective Yield [%] | Effective Selectivity [%] |
|---|---|---|---|---|
| | 346 | 97.1 | 91.9 | 94.6 |
| | 350 | 97.6 | 92.0 | 94.3 |
| A | 353 | 98.0 | 92.2 | 94.1 |
| | 357 | 98.3 | 92.2 | 93.8 |
| | 359 | 98.5 | 92.2 | 93.6 |
| | 361 | 98.7 | 92.0 | 93.2 |
| A1 | 331 | | 91.2 | |
| A2 | 373 | | 91.2 | |

[Example 8]

As shown in Table 2-11, the catalyst 6 and the catalyst 2 were packed in the order of the first layer and the second layer, and the oxidation reaction of propylene was carried out exactly under the same reaction conditions by the aging method as in Example 7. The reaction results were examined with changing the reaction bath temperature, and the results thereof are shown in Table 3-11.

[Table 2-11]

| Catalyst Type | Dilution Rate d [%] | Fill Length [cm] | CB [%/°C] | S [%/°C] | Savg [%/°C] |
|---|---|---|---|---|---|
| Catalyst 6 | 100 | 110 | 0.345 | 0.108 | 0.257 |
| Catalyst 2 | 100 | 242 | 0.590 | 0.406 | |

[Table 3-11]

| BT [°C] | | Propylene Conversion Rate [%] | Effective Yield [%] | Effective Selectivity [%] |
|---|---|---|---|---|
| | 335 | 96.9 | 91.2 | 94.1 |
| | 342 | 97.7 | 91.8 | 94.0 |
| A | 345 | 98.0 | 91.9 | 93.8 |
| | 348 | 98.3 | 91.9 | 93.5 |
| | 351 | 98.5 | 91.8 | 93.2 |
| | 353 | 98.6 | 91.8 | 93.1 |
| A1 | 331 | | 90.9 | |
| A2 | 387 | | 90.9 | |

A summary of the results is as shown in Table 4. By appropriately calculating the reaction conditions SVo to Savg and controlling the filling method so as to set the T of the invention in an appropriate range, (A2-A1) can be made wide, and a high effective yield can be obtained.

[Table 4]

| | Savg [%/°C] | SVo [/hr] | T [%·hr/°C] | A2-A1 [°C] |
|---|---|---|---|---|
| Example 1 | 0.149 | 90 | 16.6 | 36 |
| Example 2 | 0.103 | 90 | 11.4 | 55 |
| Example 3 | 0.103 | 120 | 8.6 | 28 |
| Comparative Example 1 | 0.291 | 90 | 32.3 | 22 |
| Example 4 | 0.252 | 140 | 18.0 | 34 |
| Example 5 | 0.169 | 140 | 12.1 | 58 |
| Comparative Example 2 | 0.293 | 140 | 20.9 | 22 |
| Example 6 | 0.103 | 84 | 12.3 | 74 |
| Comparative Example 3 | 0.291 | 84 | 34.6 | 13 |
| Example 7 | 0.259 | 180 | 14.4 | 42 |
| Example 8 | 0.257 | 180 | 14.3 | 57 |

[Production Example (Preparation of Catalyst)]

(Catalyst 8)

In the production of the catalyst 1, the carrier mass and the preliminarily calcined powder mass used for forming

were adjusted in such a manner that the carrying ratio defined by the above equation became 50 mass% in a powder obtained by mixing crystalline cellulose in the preliminarily calcined powder on an inert carrier (a spherical substance containing alumina and silica as main components and having a diameter of 4.5 mm). A 30 mass% glycerin aqueous solution was used as a binder, and a carried catalyst was obtained by carrying and forming into spheres having a diameter of 5.3 mm. The carried catalyst was calcined at a calcination temperature of 510°C for five hours in an air atmosphere to obtain a catalyst 8. The results of evaluation in the same manner as for the catalyst 1 and the calculated CB are shown in Table 1-8.

[Table 1-8]

| BT [°C] | Propylene Conversion Rate [%] |
|---|---|
| 340 | 20.0 |
| 360 | 25.1 |
| CB | 0.255 |

[Example 9]

In a stainless steel reactor having an inner diameter of 25.4 mm in which a jacket for circulating a melted salt as a heating medium and a thermocouple for measuring the temperatures of the catalyst layers were installed on the tube shaft, a thermowell for the thermocouple having an outer diameter of 3.2 mm was installed, and as shown in Table 2-12, the catalyst 8 and the catalyst 7 were packed in the order of the first layer and the second layer. The supplied amounts of propylene, air, water, and nitrogen were set in such a manner that the raw material molar ratio became propylene:oxygen (oxygen contained in the supplied air):water:nitrogen (nitrogen supplied separately from air) = 1:1.78:1.23:2.55. The supplied raw materials were allowed to flow in such a manner that the space velocity (SVo) of propylene became 125 $hr^{-1}$, and when 300 hours had passed after starting the reaction at a pressure at the outlet side of the reaction tube of 57 kPaG at the time of the total gas flow, the reaction bath temperature was changed, and the oxidation reaction of propylene was carried out. The reaction results were examined with changing the reaction bath temperature, and the results thereof are shown in Table 3-12.

[Table 2-12]

| Catalyst Type | Dilution Rate d [%] | Fill Length [cm] | CB [%/°C] | S [%/°C] | Savg [%/°C] |
|---|---|---|---|---|---|
| Catalyst 8 | 100 | 110 | 0.255 | 0.0935 | 0.231 |
| Catalyst 7 | 100 | 190 | 0.581 | 0.368 | |

[Table 3-12]

| BT [°C] | | Propylene Conversion Rate [%] | Effective Yield [%] | Effective Selectivity [%] |
|---|---|---|---|---|
| | 318 | 96.2 | 91.3 | 94.9 |
| | 322 | 97.0 | 92.0 | 94.8 |
| | 326 | 97.8 | 92.5 | 94.6 |
| A | 334 | 98.4 | 92.7 | 94.2 |
| | 336 | 98.6 | 92.6 | 93.9 |
| A1 | 321 | | 91.7 | |
| A2 | 361 | | 91.7 | |

[Example 10]

In a stainless steel reactor having an inner diameter of 24.9 mm in which a jacket for circulating a melted salt as a heating medium and a thermocouple for measuring the temperatures of the catalyst layers were installed on the tube shaft, a thermowell for the thermocouple having an outer diameter of 7 mm was installed, and as shown in Table 2-13, a catalyst 8-diluted layer, the catalyst 8, and the catalyst 7 were packed in the order of the first layer, the second layer, and the third layer. The supplied amounts of propylene, air, water, and nitrogen were set in such a manner that the raw material molar

ratio became propylene:oxygen (oxygen contained in the supplied air):water:nitrogen (nitrogen supplied separately from air) = 1:1.68:1.05:2.43. The supplied raw materials were allowed to flow in such a manner that the space velocity (SVo) of propylene became 100 $hr^{-1}$, and when 300 hours had passed after starting the reaction at a pressure at the outlet side of the reaction tube of 57 kPaG at the time of the total gas flow, the reaction bath temperature was changed, and the oxidation reaction of propylene was carried out. The reaction results were examined with changing the reaction bath temperature, and the results thereof are shown in Table 3-13.

[Table 2-13]

| Catalyst Type | Dilution Rate d [%] | Fill Length [cm] | CB [%/°C] | S [%/°C] | Savg [%/°C] |
|---|---|---|---|---|---|
| Catalyst 8 | 75 | 65 | 0.255 | 0.0355 | 0.140 |
| Catalyst 8 | 100 | 95 | 0.255 | 0.0692 | |
| Catalyst 7 | 100 | 190 | 0.581 | 0.315 | |

[Table 3-13]

| BT [°C] | | Propylene Conversion Rate [%] | Effective Yield [%] | Effective Selectivity [%] |
|---|---|---|---|---|
| | 318 | 96.5 | 91.1 | 94.4 |
| | 321 | 98.0 | 92.3 | 94.2 |
| | 324 | 98.5 | 92.7 | 94.2 |
| | 327 | 98.8 | 92.7 | 93.9 |
| A | 330 | 99.0 | 92.8 | 93.8 |
| | 333 | 99.1 | 92.7 | 93.5 |
| A1 | 321 | | 91.8 | |
| A2 | 355 | | 91.8 | |

[Example 11]

In a stainless steel reactor having an inner diameter of 25.2 mm in which a jacket for circulating a melted salt as a heating medium and a thermocouple for measuring the temperatures of the catalyst layers were installed on the tube shaft, a thermowell for the thermocouple having an outer diameter of 3.2 mm was installed, and as shown in Table 2-14, a catalyst 8-diluted layer, the catalyst 8, and the catalyst 7 were packed in the order of the first layer, the second layer, and the third layer. The supplied amounts of propylene, air, water, and nitrogen were set in such a manner that the raw material molar ratio became propylene:oxygen (oxygen contained in the supplied air):water:nitrogen (nitrogen supplied separately from air) = 1:1.75:1.15:2.20. The supplied raw materials were allowed to flow in such a manner that the space velocity (SVo) of propylene became 115 $hr^{-1}$, and when 300 hours had passed after starting the reaction at a pressure at the outlet side of the reaction tube of 52 kPaG at the time of the total gas flow, the reaction bath temperature was changed, and the oxidation reaction of propylene was carried out. The reaction results were examined with changing the reaction bath temperature, and the results thereof are shown in Table 3-14.

[Table 2-14]

| Catalyst Type | Dilution Rate d [%] | Fill Length [cm] | CB [%/°C] | S [%/°C] | Savg [%/°C] |
|---|---|---|---|---|---|
| Catalyst 8 | 75 | 65 | 0.255 | 0.0355 | 0.140 |
| Catalyst 8 | 100 | 95 | 0.255 | 0.0692 | |
| Catalyst 7 | 100 | 190 | 0.581 | 0.315 | |

[Table 3-14]

| BT [°C] | | Propylene Conversion Rate [%] | Effective Yield [%] | Effective Selectivity [%] |
|---|---|---|---|---|
| | 315 | 93.2 | 88.3 | 94.7 |
| | 320 | 97.4 | 92.1 | 94.6 |

(continued)

| BT [°C] | | Propylene Conversion Rate [%] | Effective Yield [%] | Effective Selectivity [%] |
|---|---|---|---|---|
| | 325 | 98.1 | 92.5 | 94.3 |
| A | 330 | 98.7 | 92.8 | 94.0 |
| | 335 | 99.0 | 92.7 | 93.6 |
| A1 | 323 | | 91.8 | |
| A2 | 370 | | 91.8 | |

[Example 12]

In a stainless steel reactor having an inner diameter of 27.8 mm in which a jacket for circulating a melted salt as a heating medium and a thermocouple for measuring the temperatures of the catalyst layers were installed on the tube shaft, a thermowell for the thermocouple having an outer diameter of 3.2 mm was installed, and as shown in Table 2-15, a catalyst 8-diluted layer, the catalyst 8, and the catalyst 7 were packed in the order of the first layer, the second layer, and the third layer. The supplied amounts of propylene, air, water, and nitrogen were set in such a manner that the raw material molar ratio became propylene:oxygen (oxygen contained in the supplied air):water:nitrogen (nitrogen supplied separately from air) = 1:1.63:0.63:3.36. The supplied raw materials were allowed to flow in such a manner that the space velocity (SVo) of propylene became 76 $hr^{-1}$, and when 300 hours had passed after starting the reaction at a pressure at the outlet side of the reaction tube of 43 kPaG at the time of the total gas flow, the reaction bath temperature was changed, and the oxidation reaction of propylene was carried out. The reaction results were examined with changing the reaction bath temperature, and the results thereof are shown in Table 3-15.

[Table 2-15]

| Catalyst Type | Dilution Rate d [%] | Fill Length [cm] | CB [%/°C] | S [%/°C] | Savg [%/°C] |
|---|---|---|---|---|---|
| Catalyst 8 | 75 | 65 | 0.255 | 0.0377 | 0.140 |
| Catalyst 8 | 100 | 95 | 0.255 | 0.0657 | |
| Catalyst 7 | 100 | 180 | 0.581 | 0.317 | |

[Table 3-15]

| BT [°C] | | Propylene Conversion Rate [%] | Effective Yield [%] | Effective Selectivity [%] |
|---|---|---|---|---|
| | 309 | 92.9 | 87.7 | 94.4 |
| | 312 | 97.8 | 92.2 | 94.2 |
| | 315 | 98.5 | 92.7 | 94.1 |
| | 318 | 98.9 | 92.8 | 93.8 |
| A | 321 | 99.1 | 92.9 | 93.7 |
| | 325 | 99.3 | 92.7 | 93.3 |
| A1 | 315 | | 91.9 | |
| A2 | 346 | | 91.9 | |

A summary of the results of Example 9 to Example 12 is as shown in Table 5. It can be seen again that, by appropriately calculating the reaction conditions SVo to Savg and controlling the filling method so as to set the production parameter T of the invention in an appropriate range, (A2-A1) can be made wide, and a high effective yield can be obtained.

[Table 5]

| | Savg [%/°C] | SVo [/hr] | T [%·hr/°C] | A2-A1 [°C] |
|---|---|---|---|---|
| Example 9 | 0.231 | 125 | 18.5 | 40 |
| Example 10 | 0.140 | 100 | 14.0 | 34 |

(continued)

| | Savg [%/°C] | SVo [/hr] | T [%·hr/°C] | A2-A1 [°C] |
|---|---|---|---|---|
| Example 11 | 0.140 | 115 | 12.2 | 47 |
| Example 12 | 0.140 | 76 | 18.4 | 31 |

The present application is based on Japanese Patent Application No. 2023-191217 filed on November 9, 2023, contents of which are incorporated herein by reference.

INDUSTRIAL APPLICABILITY

According to the invention of the present application, in the case of producing a corresponding unsaturated aldehyde and unsaturated carboxylic acid using an alkene or an alcohol capable of producing an alkene by an intramolecular dehydration reaction thereof as a raw material, a high yield can be achieved in a wide reaction bath temperature range even in an industrial plant, and wide (A2-A1) means that the high yield can be maintained in a wider reaction bath temperature range. That is, even when the catalytic activity decreases and the reaction bath temperature increases with time due to plant operation for a long period of time, according to the invention, a high yield can be maintained over a long period of time.

## Claims

1. A method for producing an unsaturated aldehyde or an unsaturated carboxylic acid, comprising:

   partially oxidizing an alkene to produce a corresponding unsaturated aldehyde or unsaturated carboxylic acid using a fixed-bed multi-tubular reactor,
   wherein a production parameter T satisfies the following formula (I):

   $$T \leq 20.5 \quad (I)$$

   where, T is calculated from an average filling parameter Savg and a space velocity SVo of a raw material according to an equation (IV),

   $$T\ (\%\cdot hr/°C) = Savg\ (\%/°C) \div SVo\ (/hr) \times 10000 \quad (IV),$$

   Savg is calculated from an arithmetic mean of filling parameters Si of all of catalyst-packed layers according to an equation (III),

   $$Savg\ (\%/°C) = (S1\ (\%/°C) + \cdots + Sn\ (\%/°C)) \div n \quad (III),$$

   Si is calculated from a temperature sensitivity parameter CBi, a dilution rate di, and a fill length ratio Li of a catalyst in i-th layer according to an equation (II),

   $$Si\ (\%/°C) = CBi\ (\%/°C) \times di\ (\%) \div 100 \times Li \quad (II),$$

   CBi is a parameter relating to a temperature sensitivity of the catalyst of the i-th layer and is a rate of a change in a raw material conversion rate with respect to a reaction bath temperature; n is the total number of catalyst layers in a reaction tube; an index i is a natural number of 1 to n; and the catalyst layers do not include any inert layer which is filled intentionally only with an inert substance.

2. The method for producing an unsaturated aldehyde or an unsaturated carboxylic acid according to claim 1, wherein the production parameter T satisfies the following formula (V):

   $$9.5 \leq T \leq 16.5 \quad (V).$$

3. The method for producing an unsaturated aldehyde or an unsaturated carboxylic acid according to claim 1 or 2,

wherein the average filling parameter Savg satisfies the following formula (VI):

$$0.090 \leq Savg \leq 0.30 \quad (VI).$$

**4.** The method for producing an unsaturated aldehyde or an unsaturated carboxylic acid according to claim 1 or 2, wherein a catalytically active component contained in a catalyst layer closest to an inlet of a raw material gas for reaction has a composition represented by the following formula (1-1):

$$Mo_{a1}Bi_{b1}Ni_{c1}Co_{d1}Fe_{e1}X_{f1}Cs_{g1}Z_{h1}O_{i1} \quad (1\text{-}1)$$

where, Mo, Bi, Ni, Co, Fe, and Cs respectively represent molybdenum, bismuth, nickel, cobalt, iron, and cesium, X is at least one element selected from tungsten, antimony, tin, zinc, chromium, manganese, magnesium, silicon, aluminum, cerium, and titanium, Z belongs to Group 1 to Group 16 in the periodic table and means at least one element selected from elements other than the above Mo, Bi, Ni, Co, Fe, Cs, and X, a1, b1, c1, d1, e1, f1, g1, h1, and i1 respectively represent the numbers of atoms of molybdenum, bismuth, nickel, cobalt, iron, X, Cs, Z, and oxygen, when $a1 = 12$, $0 < b1 \leq 7.0$, $0 \leq c1 \leq 10$, $0 < d1 \leq 10$, $0 < c1+d1 \leq 20$, $0 < e1 \leq 5.0$, $0 \leq f1 \leq 2.0$, $0 < g1 \leq 3.0$, and $0 \leq h1 \leq 5.0$ are satisfied, and i1 is a value determined by the oxidation state of each element.

**5.** The method for producing an unsaturated aldehyde or an unsaturated carboxylic acid according to claim 1 or 2, wherein a catalytically active component contained in a catalyst layer closest to an outlet of a raw material gas for reaction has a composition represented by the following formula (1-2):

$$Mo_{a2}Bi_{b2}Ni_{c2}Co_{d2}Fe_{e2}X_{f2}K_{g2}Z_{h2}O_{i2} \quad (1\text{-}2)$$

where, Mo, Bi, Ni, Co, Fe, and K respectively represent molybdenum, bismuth, nickel, cobalt, iron, and potassium, X is at least one element selected from tungsten, antimony, tin, zinc, chromium, manganese, magnesium, silicon, aluminum, cerium, and titanium, Z belongs to Group 1 to Group 16 in the periodic table and means at least one element selected from elements other than the above Mo, Bi, Ni, Co, Fe, K, and X, a2, b2, c2, d2, e2, f2, g2, h2, and i2 respectively represent the numbers of atoms of molybdenum, bismuth, nickel, cobalt, iron, X, K, Z, and oxygen, when $a2 = 12$, $0 < b2 \leq 7.0$, $0 \leq c2 \leq 10$, $0 < d2 \leq 10$, $0 < c2 + d2 \leq 20$, $0 < e2 \leq 5.0$, $0 \leq f2 \leq 2.0$, $0 \leq g2 \leq 3.0$, and $0 \leq h2 \leq 5.0$ are satisfied, and i2 is a value determined by the oxidation state of each element.

**6.** The method for producing an unsaturated aldehyde or an unsaturated carboxylic acid according to claim 1 or 2, wherein a plant is operated at a reaction bath temperature of 310°C or higher and 390°C or lower.

**7.** The method for producing an unsaturated aldehyde or an unsaturated carboxylic acid according to claim 1 or 2, wherein a volume ratio (oxygen/alkene) of an alkene and an oxygen contained in a raw material gas for reaction is 1.0 or more and 1.9 or less.

**8.** The method for producing an unsaturated aldehyde or an unsaturated carboxylic acid according to claim 1 or 2,

wherein n layers (n is 2 or more) of catalyst layers are provided in a gas flow direction of the reaction tube, and L/Ln is 0.1 or more and 1.0 or less, when a sum of fill lengths of a first to n-1-th catalyst layers counted from a reaction gas inlet side is L, and a fill length of an n-th catalyst layer counted from the reaction gas inlet side is Ln.

**INTERNATIONAL SEARCH REPORT**

International application No. **PCT/JP2024/038105**

**A. CLASSIFICATION OF SUBJECT MATTER**

***C07C 45/35***(2006.01)i; ***B01J 23/887***(2006.01)i; ***C07B 61/00***(2006.01)i; ***C07C 47/22***(2006.01)i; ***C07C 51/215***(2006.01)i; ***C07C 51/235***(2006.01)i; ***C07C 57/05***(2006.01)i
FI: C07C45/35; C07C47/22 A; C07C57/05; B01J23/887 Z; C07C51/215; C07C51/235; C07B61/00 300

According to International Patent Classification (IPC) or to both national classification and IPC

**B. FIELDS SEARCHED**

Minimum documentation searched (classification system followed by classification symbols)

C07C45/35; B01J23/887; C07B61/00; C07C47/22; C07C51/215; C07C51/235; C07C57/05

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| X<br>Y | JP 2018-140993 A (NIPPON KAYAKU CO., LTD.) 13 September 2018 (2018-09-13) claims, examples, paragraphs [0006], [0015] | 1-8<br>1-8 |
| X<br>Y | WO 2015/008814 A1 (NIPPON KAYAKU CO., LTD.) 22 January 2015 (2015-01-22) claims, examples, paragraph [0027] | 1-8<br>1-8 |
| Y | JP 2023-81301 A (NIPPON KAYAKU CO., LTD.) 09 June 2023 (2023-06-09) claims, examples | 1-8 |

☐ Further documents are listed in the continuation of Box C.   ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **29 November 2024** | **17 December 2024** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (July 2022)

**INTERNATIONAL SEARCH REPORT**
**Information on patent family members**

International application No.
**PCT/JP2024/038105**

| Patent document cited in search report | Publication date (day/month/year) | Patent family member(s) | Publication date (day/month/year) |
|---|---|---|---|
| JP 2018-140993 A | 13 September 2018 | US 2013/0310604 A1<br>claims, examples, paragraph [0024]<br>EP 2671862 A1<br>CN 102627537 A<br>CN 103347845 A | |
| WO 2015/008814 A1 | 22 January 2015 | US 2016/0145180 A1<br>claims, examples, paragraph [0040]<br>EP 3023405 A1<br>KR 10-2016-0014666 A<br>CN 105392761 A | |
| JP 2023-81301 A | 09 June 2023 | (Family: none) | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 7209901 B
- JP 2001328951 A
- JP 6694884 B
- JP 6912153 B
- JP 6199972 B
- JP 2023191217 A